# EUROPEAN PATENT APPLICATION

(11) **EP 0 661 284 A1**
(43) Date of publication of application: **05.07.1995**
(21) Application number: 93919673.9
(22) Date of filing: 16.09.1993
(51) Int. Cl.: C07D 495/04, C07D 495/14, A61K 31/55

(54) **THIENODIAZEPINE COMPOUND AND MEDICINAL USE THEREOF**

(30) Priority: 18.09.1992 WO PCT/JP92/01198
(71) Applicant: YOSHITOMI PHARMACEUTICAL INDUSTRIES, LTD., Osaka-shi Osaka 541 (JP)
(72) Inventor: MORIWAKI, Minoru, Yoshitomi Pharm. Industries Ltd., Yoshitomimachi, Chikujo-gun, Fukuok 871 (JP); KITANI, Hiroyuki, Yoshitomi Pharm. Industries Ltd., Yoshitomimachi, Chikujo-gun, Fukuoka 871 (JP); EHARA, Syuji, Yoshitomi Pharm. Industries Ltd., Yoshitomimachi, Chikujo-gun, Fukuoka 871 (JP); YASUMATSU, Hiroshi, Yoshitomi Pharm.Industries Ltd, Yoshitomachi, Chikujo-gun, Fukuoka 871 (JP); KOMATSU, Hirotsugu, Yoshitomi Pharm.Industries Ltd, Iruma-shi, Saitama 358 ama 358 (JP)
(74) Representative: von Kreisler, Alek, Dipl.-Chem.
(86) International application number: PCT/JP93/01329
(87) International publication number: WO 94/06802

(57) **Abstract**

A thienodiazepine compound represented by general formula (I), a salt thereof, and a medicinal use thereof, wherein R³ represents a group represented by any of the following formulae: (1), -(CH₂)aN(R⁴)COR⁷ (2), -(CH₂)aN(R⁸)SO₂R⁹ (3), -(CH₂)aN(R⁸)COOR¹⁰ (4), -(CH₂)aOCON(R¹¹)(R¹²) (5), -(CH₂)aCON(R¹³(R¹⁴) (6), -(CH₂)aOCOR¹⁵ (7), -(CH₂)aOSO₂R¹⁶ (8), -(CH₂)aCOR¹⁷ (9) and -(CH₂aS(O)ₙR¹⁸ (10), a represents an integer of 0, 1 to 6 ; Z represents oxygen or sulfur ; and X represents oxygen or sulfur, provided that X and Y may be combined together to represent = N-N = C(R²⁰)- or = N-CH = C(R²⁰)-. This compound has CCK antagonism, gastrin antagonism and cell fusion inhibitor activity, thus being useful as a medicine.

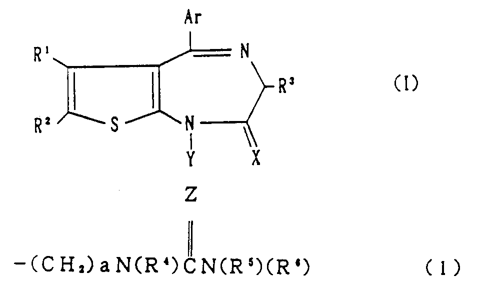

## Description

### [Technical Field]

The present invention relates to novel thienodiazepine compounds having superior antagonistic action on cholecystokinin and gastrin, as well as superior inhibitory action on cell adhesion, salts thereof, and pharmaceutical use thereof.

### [Background Art]

The cholecystokinin (also referred to as CCK) is a neuropeptide consisting of 33 amino acids, and CCK-8 which consists of 8 amino acids at the C terminus also shows activity. The gastrin consists of 34 amino acids, and pentagastrin which consists of 5 amino acids at the C terminus also shows activity. The amino acid sequence of the pentagastrin is identical to that at the N terminus of CCK. The both and their receptors exist in gastrointestinal tissues and the central nervous system, and are considered to be concerned with the control of pancreatic enzyme secretion and gastric acid secretion, and emotions such as anxiety.

Compounds which exhibit antagonistic action on these CCK and gastrin are considered to be effective in the prophylaxis and therapy of diseases such as pancreatic disorders and gastrointestinal ulcers, and as an anxiolytic agent, and a number of antagonistic substances have been studied so far.

As an antagonistic substance to CCK, benzotripto is known [Proc. Natl. Acad. Sci. U.S.A., vol. 78, p. 6304 (1981)], and proglumide is known as an antagonistic substance to gastrin [J. Med. Chem., vol. 27, p. 1597 (1984)]. However, their actions are relatively weak, and compounds exhibiting higher activities and superior safety have been desired.

Besides, peptide antagonistic substances are not entirely satisfactory in that the durability of their actions is short and in that they are unstable and poorly absorbed.

It has been recently clarified that various cell adhesion molecules are deeply concerned with the onset and progress of certain inflammatory diseases and allergic diseases. Hence, compounds having inhibitory action on cell adhesion are expected to make superior anti inflammatory drugs or antiallergic drugs. For example, Proc. Natl. Acad. Sci. U.S.A., vol. 88, pp 355-359 (1991) reports that N-(fluorenyl-9-methoxycarbonyl)amino acids suppress inflammatory reactions in various animal models by inhibiting adhesion of leukocytes, and an extract from American Federation for Clinical Research Annual Meeting, May 6, 1991, reports that the same series of compounds inhibit leukocyte adhesion to vascular endothelial cells, by suppressing the expression of adhesion molecules such as CD18 on leukocytes.

Moreover, International Publication No. WO 89/05812 and Japanese Patent Unexamined Publication No. 223290/1991 disclose a thienodiazepine compound having an antagonistic action on CCK and gastrin.

The above-mentioned CCK antagonistic drugs and gastrin antagonistic drugs heretofore reported do not exhibit sufficient effects and the development of a compound having more superior action is desired. In addition, the development of a superior antiinflammatory drug or antiallergic drug having an inhibitory action on adhesion of leukocytes to vascular endothelial cells is also desired.

### [Disclosure of the Invention]

The present inventors have conducted intensive studies with the aim of developing a more superior CCK antagonistic drug, gastrin antagonistic drug and/or cell adhesion-inhibitory drug, and found that a certain kind of thienodiazepine compound has a superior CCK antagonistic action, gastrin antagonistic action or inhibitory action on adhesion of leukocytes to vascular endothelial cells, which resulted in the completion of the invention.

That is, the present invention relates to thienodiazepine compounds of the formula 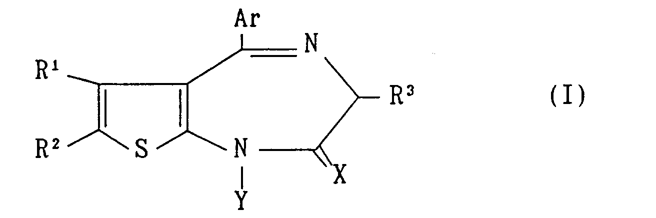
wherein
- Ar: is an optionally substituted aryl or an optionally substituted heteroaryl;
- R¹: is a hydrogen, a halogen, an alkyl having 1 to 20 carbon atoms or an optionally substituted aralkyl;
- R²: is a hydrogen, a halogen, an alkyl having 1 to 20 carbon atoms, an optionally substituted aralkyl, an alkenyl having 2 to 20 carbon atoms or an alkynyl having 2 to 20 carbon atoms; or
- R¹ and R²: may combinedly together form an optionally substituted 5 to 7-membered ring, or an optionally substituted 5 to 7-membered heterocyclic ring together with a hetero atom;
- R³: is a group of the formula 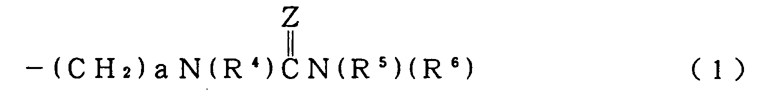
wherein a is 0 or an integer of 1-6, Z is an oxygen atom or a sulfur atom, R⁴ is a hydrogen, an alkyl having 1 to 20 carbon atoms or an optionally substituted aralkyl, R⁵ and R⁶ are the same or different and each is a hydrogen, an alkyl having 1 to 20 carbon atoms, an optionally substituted cycloalkyl, an optionally substituted cycloalkylalkyl, an optionally substituted aryl, an optionally substituted aralkyl, an optionally substituted heteroaryl or an optionally substituted heteroarylalkyl,
a group of the formula

-(CH₂)aN(R⁴)COR⁷ (2)

wherein a is 0 or an integer of 1-6, R⁴ is a hydrogen, an alkyl having 1 to 20 carbon atoms or an optionally substituted aralkyl, R⁷ is an alkyl having 1 to 20 carbon atoms, an alkenyl having 2 to 20 carbon atoms, alkynyl having 2 to 20 carbon atoms, an optionally substituted aryl, an optionally substituted heteroaryl, an optionally substituted aralkyl or an optionally substituted heteroarylalkyl, or
a group of the formula selected from the formulas:

-(CH₂)aN(R⁸)SO₂R⁹ (3)

-(CH₂)aN(R⁸)COOR¹⁰ (4)

-(CH₂)aOCON(R¹¹)(R¹²) (5)

-(CH₂)aCON(R¹³)(R¹⁴) (6)

-(CH₂)aOCOR¹⁵ (7)

-(CH₂)aOSO₂R¹⁶ (8)

-(CH₂)aCOR¹⁷ (9) and

-(CH₂)aS(O)ₙR¹⁸ (10)

wherein a is 0 or an integer of 1-6, R⁸ is a hydrogen, an alkyl having 1 to 20 carbon atoms or an optionally substituted aralkyl, R⁹ and R¹⁰ are each an alkyl having 1 to 20 carbon atoms, alkenyl having 2 to 20 carbon atoms, alkynyl having 2 to 20 carbon atoms, an optionally substituted cycloalkyl, an optionally substituted cycloalkylalkyl, an optionally substituted aryl, an optionally substituted heteroaryl, an optionally substituted aralkyl or an optionally substituted heteroarylalkyl, R¹¹, R¹², R¹³ and R¹⁴ are the same or different and each is a hydrogen, an alkyl having 1 to 20 carbon atoms, an alkenyl having 2 to 20 carbon atoms, an alkynyl having 2 to 20 carbon atoms, an optionally substituted aryl, an optionally substituted heteroaryl, an optionally substituted aralkyl or an optionally substituted heteroarylalkyl, R¹⁵, R¹⁶, R¹⁷ and R¹⁸ are each an alkyl having 1 to 20 carbon atoms, an optionally substituted aryl, an optionally substituted heteroaryl, an optionally substituted aralkyl or an optionally substituted heteroarylalkyl, and n is 0, 1 or 2;
- X: is an oxygen atom or a sulfur atom; and
- Y: is a hydrogen, an alkyl having 1 to 20 carbon atoms, an alkenyl having 2 to 20 carbon atoms, an alkynyl having 2 to 20 carbon atoms, an optionally substituted aralkyl, an optionally substituted heteroarylalkyl or a group of the formula

-(CH₂)bCOOR¹⁹

wherein R¹⁹ is a hydrogen, an alkyl having 1 to 20 carbon atoms, an alkenyl having 2 to 20 carbon atoms or an optionally substituted aralkyl and b is an integer of 1 to 6; or X and Y may combinedly form a group of the formula:

=N-N=C(R²⁰)- or =N-CH=C(R²⁰)-

wherein R²⁰ is a hydrogen, a halogen, an alkyl having 1 to 20 carbon atoms, an alkenyl having 2 to 20 carbon atoms, an alkynyl having 2 to 20 carbon atoms, an optionally substituted cycloalkyl, an optionally substituted cycloalkylalkyl, an optionally substituted aryl, an optionally substituted heteroaryl, an optionally substituted aralkyl, an optionally substituted heteroarylalkyl, or a group of the formula:

-(CH₂)bCOOR²⁰'

wherein R²⁰' is a hydrogen, an alkyl having 1 to 20 carbon atoms, an alkenyl having 2 to 20 carbon atoms or an optionally substituted aralkyl and b is an integer of 1 to 6;
with the proviso that
when R³ is a group of the above-mentioned formula (1) or (2), X and Y combinedly form a group of the formula:

=N-N=C(R²¹)- or =N-CH=C(R²¹)-

wherein R²¹ is an optionally substituted cycloalkyl, an optionally substituted cycloalkylalkyl, an optionally substituted aryl, an optionally substituted heteroaryl or an optionally substituted heteroarylalkyl; and
when R³ is a group of the above-mentioned formula (7), R¹, R² and Y are each a hydrogen or an alkyl having 1 to 4 carbon atoms, X is an oxygen atom, Ar is a phenyl substituted by halogen and a is 0, R¹⁵ is an alkyl having 5 to 20 carbon atoms, an optionally substituted aryl, an optionally substituted heteroaryl, an optionally substituted aralkyl or an optionally substituted heteroarylalkyl;
and salts thereof.

Specific examples of the compounds of the formula (I) include compounds of the formula 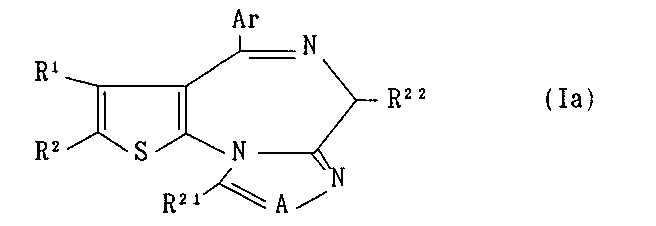
wherein Ar, R¹, R² and R²¹ are as defined above, A is a nitrogen atom or CH and R²² is a group of the formula 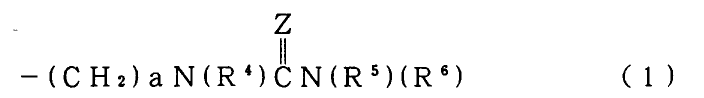
wherein each symbol is as defined above, or a group of the formula

-(CH₂)aN(R⁴)COR⁷ (2)

wherein each symbol is as defined above, salts thereof, compounds of the formula 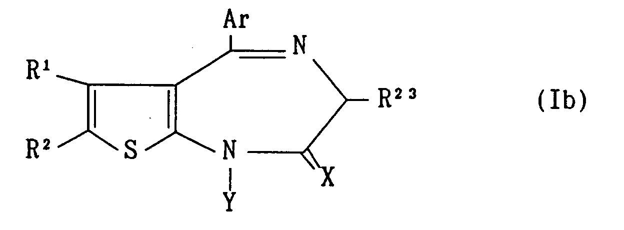
wherein Ar, R¹, R², X and Y are as defined above and R²³ is a group of the formula selected from the formulas:

-(CH₂)aN(R⁸)SO₂R⁹ (3)

-(CH₂)aN(R⁸)COOR¹⁰ (4)

-(CH₂)aOCON(R¹¹)(R¹²) (5)

-(CH₂)aCON(R¹³)(R¹⁴) (6)

-(CH₂)aOCOR¹⁵ (7)

-(CH₂)aOSO₂R¹⁶ (8)

-(CH₂)aCOR¹⁷ (9) and

-(CH₂)aS(O)ₙR¹⁸ (10)

wherein each symbol is as defined above, provided that when R²³ is a group of the above-mentioned formula (7), R¹, R² and Y are each a hydrogen or an alkyl having 1 to 4 carbon atoms, X is an oxygen atom, Ar is a phenyl substituted by halogen and a is 0, R¹⁵ is an alkyl having 5 to 20 carbon atoms, an optionally substituted aryl, an optionally substituted heteroaryl, an optionally substituted aralkyl or an optionally substituted heteroarylalkyl, and salts thereof.

The preferable compounds of the formula (Ia) include N-(4-(2-chlorophenyl)-9-cyclohexyl-2-ethyl-6H-thieno[3,2-f][1,2,4]triazolo[4,3-a][1,4]diazepin-6-yl)-N'-(4-methoxyphenyl)urea,
N-(2-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(4-(2-chlorophenyl)-9-cyclohexyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(2-methylphenyl)urea,
N-(4-(2-chlorophenyl)-9-cyclopentyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclopentyl-2-ethyl-6H-thieno[3,2-f][1,2,4]triazolo[4,3-a][1,4]diazepin-6-yl)-N'-(4-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(4-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(3-methoxyphenyl) urea,
N-(4-(2-chlorophenyl)-9-cyclopentyl-2-ethyl-6H-thieno[3,2-f][1,2,4]triazolo[4,3-a][1,4]diazepin-6-yl)-N'-(2-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(2-methoxyphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(4-methylphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(phenyl)urea,
N-(4-(2-chlorophenyl)-2-ethyl-9-phenyl-6H-thieno[3,2-f][1,2,4]triazolo[4,3-a][1,4]diazepin-6-yl)-N'-(2-methoxyphenyl)urea,
N-(4-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(3-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(4-(2-chlorophenyl)-9-cyclohexyl-2-ethyl-6H-thieno[3,2-f][1,2,4]triazolo[4,3-a][1,4]diazepin-6-yl)-N'-(3-methoxyphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(2-methoxyphenyl)urea,
N-(4-(2-chlorophenyl)-2-ethyl-9-phenyl-6H-thieno[3,2-f][1,2,4]triazolo[4,3-a][1,4]diazepin-6-yl)-N'-(4-methoxyphenyl)urea,
N-(4-(2-chlorophenyl)-9-cycloheptyl-2-ethyl-6H-thieno[3,2-f][1,2,4]triazolo[4,3-a][1,4]diazepin-6-yl)-N'-(4-methoxyphenyl)urea,
N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea,
N-(9-(1-adamantyl)-4-(2-chlorophenyl)-2-ethyl-6H-thieno[3,2-f][1,2,4]triazolo[4,3-a][1,4]diazepin-6-yl)-N'-(2-chlorophenyl)urea,
(+)-N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea and salts thereof.

The preferable compounds of the formula (Ib) include N-phenyl-(4-(4-chlorophenyl)-2-ethyl-9-methyl-6H-thieno[3,2-f][1,2,4]triazolo[4,3-a][1,4]diazepin-6-yl)acetamide,
N-(3-methylphenyl)-(4-(4-chlorophenyl)-2-ethyl-9-methyl-6H-thieno[3,2-f][1,2,4]triazolo[4,3-a][1,4]diazepin-6-yl)acetamide,
N-(3-chlorophenyl)-(4-(4-chlorophenyl)-2-ethyl-9-methyl-6H-thieno[3,2-f][1,2,4]triazolo[4,3-a][1,4]diazepin-6-yl)acetamide,
N-(2-methoxyphenyl)-(4-(4-chlorophenyl)-2-ethyl-9-methyl-6H-thieno[3,2-f][1,2,4]triazolo[4,3-a][1,4]diazepin-6-yl)acetamide,
N-(3-methoxyphenyl)-(4-(4-chlorophenyl)-2-ethyl-9-methyl-6H-thieno[3,2-f][1,2,4]triazolo[4,3-a][1,4]diazepin-6-yl)acetamide,
N-(4-(4-chlorophenyl)-2-ethyl-9-methyl-6H-thieno[3,2-f][1,2,4]triazolo[4,3-a][1,4]diazepin-6-yl)-p-toluenesulfonamide,
N-(4-(2-chlorophenyl)-2-(4-isobutylphenyl)ethyl-9-methyl-6H-thieno[3,2-f][1,2,4]triazolo[4,3-a][1,4]diazepin-6-yl)-p-toluenesulfonamide,
(5-(2-chlorophenyl)-7-ethyl-1-methyl-2-oxo-1,3-dihydro-2H-thieno[2,3-e][1,4]diazepin-3-yl)-N-(3-methylphenyl)carbamate,
(4-(2-chlorophenyl)-2-ethyl-9-methyl-6H-thieno[3,2-f][1,2,4]triazolo[4,3-a][1,4]diazepin-6-yl)-N-(3-methylphenyl)carbamate and salts thereof.

The present invention also provides a pharmaceutical composition comprising a therapeutically effective amount of a compound of the formula (I), (Ia) or (Ib) and pharmaceutically acceptable additives, and thienodiazepine compounds of the formula 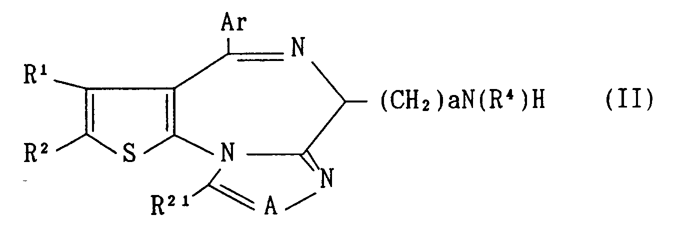
wherein A is a nitrogen atom or CH and other symbols are as defined above, and salts thereof.

With regard to the formula (I), aryl is exemplified by phenyl, 1-naphthyl and 2-naphthyl, which optionally have, on the aromatic ring, 1 to 3 substituents selected from halogen, alkyl having 1 to 6 carbon atoms, alkoxy having 1 to 6 carbon atoms, trifluoromethyl, nitro, amino, cyano and hydroxyl, with preference given to phenyl.

With regard to heteroaryl, the hetero atom constituting the ring is nitrogen, oxygen, sulfur and the like. The heteroaryl may have, on the ring, 1 to 3 substituents selected from halogen, alkyl having 1 to 6 carbon atoms, alkoxy having 1 to 6 carbon atoms, trifluoromethyl, nitro, amino, cyano and hydroxyl, and is exemplified by pyridyl (e.g. 2-pyridyl, 3-pyridyl and 4-pyridyl), quinolyl (e.g. 2-quinolyl and 3-quinolyl), indolyl (e.g. 2-indolyl and 3-indolyl), thienyl (e.g. 2-thienyl and 3-thienyl), furyl (e.g. 2-furyl and 3-furyl), benzofuranyl (e.g. 2-benzofuranyl and 3-benzofuranyl), 1H-benzimidazol-2-yl, 2-thiazolyl and 2-benzothiazolyl, with particular preference given to pyridyl, 2-thiazolyl and 2-benzothiazolyl.

Halogen means chlorine, fluorine, bromine and iodine.

Alkyl having 1 to 20 carbon atoms may be linear or branched, and is exemplified by methyl, ethyl, propyl, isopropyl, butyl, isobutyl, tert-butyl, pentyl, isopentyl, tert-pentyl, hexyl, octyl, 2-ethylhexyl, 2,2-diethyloctyl, 1,1,3,3-tetramethylbutyl, nonyl, decyl, dodecyl, tetradecyl, octadecyl and icocyl. The alkyl preferably has 1 to 6, particularly preferably 1 to 4, carbon atoms.

Optionally substituted aralkyl is that where the alkyl moiety has 1 to 6, preferably 1 to 4, carbon atoms, and may have, on the aromatic ring, 1 to 3 substituents selected from halogen, alkyl having 1 to 6 carbon atoms, alkoxy having 1 to 6 carbon atoms, trifluoromethyl, nitro, amino, cyano and hydroxyl. Examples thereof include benzyl, 1-phenylethyl, 2-phenylethyl, 3-phenylpropyl, 4-phenylbutyl, 5-phenylpentyl, 6-phenylhexyl, 1-naphthylmethyl, 2-naphthylmethyl, diphenylmethyl and 4-phenyl-3-methylbutyl, with particular preference given to benzyl and 2-phenylethyl.

Examples of alkenyl having 2 to 20 carbon atoms include vinyl, propenyl, 2-methyl-1-propenyl, 3-methyl-1-butenyl, 2,3-dimethyl-1-butenyl, 3,4,5-trimethyl-1-butenyl, 3-butenyl, 3-hexenyl, 5-dodecenyl, 6-ethyl-3-decenyl, 11,11-dimethyl-7-tetradecenyl, 14-octadecenyl and 8-icocenyl. The alkenyl preferably has 2 to 8, particularly preferably 2 to 4, carbon atoms.

Examples of alkynyl having 2 to 20 carbon atoms include 1-propynyl, 3-methyl-1-butynyl, 1,4-dimethyl-1-hexynyl, ethynyl, propargyl, 3-hexynyl, 3,4-diethyl-1-octynyl, 5-dodecynyl, 6-ethyl-3-decynyl, 11,11-dimethyl-7-tetradecynyl, 14-octadecynyl and 8-icocynyl. The alkynyl preferably has 2 to 8, particularly preferably 2 to 4, carbon atoms.

Optionally substituted cycloalkyl has 3 to 10 carbon atoms, and is exemplified by cyclopropyl, 2,3-dimethylcyclopropyl, cyclobutyl, 3-methylcyclobutyl, cyclopentyl, 3,4-dimethylcyclopentyl, cyclohexyl, 4-methylcyclohexyl, 4-tert-butylcyclohexyl, cycloheptyl, cyclooctyl, norbornyl, 1-adamantyl, bicyclo[3.3.0]octan-1-yl and bicyclo[3.3.1]nonan-9-yl, with preference given to cyclopropyl, cyclopentyl, cyclohexyl, 4-tert-butylcyclohexyl, cycloheptyl and 1-adamantyl.

Optionally substituted cycloalkylalkyl is that where the cycloalkyl moiety has 3 to 10 carbon atoms and the alkyl moiety has 1 to 6, preferably 1 to 3, carbon atoms, and is exemplified by cyclopropylmethyl, 2,3-dimethylcyclopropyl, cyclobutylmethyl, 3-methylcyclobutylmethyl, cyclopentylmethyl, 3,4-dimethylcyclopentylmethyl, cyclohexylmethyl, 4-methylcyclohexylmethyl, cycloheptylmethyl, cyclooctylmethyl, 2-cyclohexylethyl, 3-cyclohexylpropyl, norbornylmethyl, 1-adamantylmethyl, bicyclo[3.3.0]octan-1-ylmethyl and bicyclo[3.3.1]nonan-9-ylmethyl, with particular preference given to cyclohexylmethyl.

Optionally substituted heteroarylalkyl may have, on the ring, 1 to 3 substituents selected from halogen, alkyl having 1 to 6 carbon atoms, alkoxy having 1 to 6 carbon atoms, trifluoromethyl, nitro, amino, cyano and hydroxyl. The alkyl moiety thereof has 1 to 4, preferably 1 or 2, carbon atoms, and the hetero atom constituting the ring is nitrogen, oxygen or sulfur. Examples thereof include pyridyl(2-pyridyl, 3-pyridyl or 4-pyridyl)methyl, quinolyl(2-quinolyl or 3-quinolyl)methyl, indolyl(2-indolyl or 3-indolyl)methyl, thienyl(2-thienyl or 3-thienyl)methyl, furyl(2-furyl or 3-furyl)methyl, benzofuranyl(2-benzofuranyl or 3-benzofuranyl)methyl, 1H-benzimidazol-2-ylmethyl, 2-benzothiazolylmethyl, 2-(2-thienyl)ethyl and 2-(2-furyl)ethyl.

The optionally substituted 5 to 7-membered ring formed by R¹ and R² in combination and the optionally substituted 5 to 7-membered heterocyclic ring formed by R¹ and R² together with hetero atom are exemplified by cyclopentene, cyclopentadiene, cyclohexene, cyclohexadiene, cycloheptene, cycloheptadiene, cycloheptatriene, benzene, dihydropyran, dihydrothiapyran and tetrahydropyridine, and examples of the substituent include alkyl such as methyl and ethyl, benzyl and aralkyl such as 2-phenylethyl.

The halogen as a substituent means chlorine, fluorine, bromine or iodine, and alkyl having 1 to 6 carbon atom as a substituent is exemplified by methyl, ethyl, propyl, isopropyl, butyl, isobutyl, tert-butyl, pentyl, isopentyl, tert-pentyl and hexyl. Alkoxy having 1 to 6 carbon atoms is, for example, methoxy, ethoxy, propoxy, isopropoxy, butoxy, isobutoxy, tert-butoxy, pentyloxy, isopentyloxy, tert-pentyloxy or hexyloxy. Alkyl and alkoxy particularly preferably have 1 to 4 carbon atoms.

The salts of the compounds of the formula (I) are, for example, acid addition salts with inorganic acid or organic acid, and salts with inorganic base, organic base or amino acid. In view of the object of the present invention, these salts are preferably nontoxic.

The compounds of the formulas (I), (Ia) and (Ib) have at least one chiral carbon atom. Hence, the racemates, optically active compounds and diastereomers thereof are all encompassed in the present invention.

Of the compounds of the formula (I), the compounds of (Ia) can be produced by, for example, adding isocyanate or isothiocyanate of the formula

R²⁴NCZ (III)

wherein R²⁴ is R⁵ or R⁶ and R⁵, R⁶ and Z are as defined above, to a compound of the formula 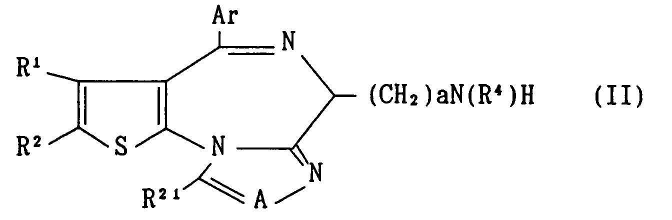
wherein each symbol is as defined above, or by condensing the compound (II) with a compound of the formula

R⁷COW (IV)

wherein R⁷ is as defined above, and W is a leaving group such as hydroxyl, halogen, ester (e.g. pentachlorophenoxy and p-nitrophenoxy) and thioester (e.g. phenylthio and 2,6-dimethylpyridine-4-thio).

The addition reaction between the compound of the formula (II) and the compound of the formula (III) is carried out in a suitable solvent which does not inhibit the instant reaction. Examples of the solvent include organic solvents such as tetrahydrofuran, diethyl ether, diisopropyl ether, dioxane, dichloromethane, chloroform, carbon tetrachloride, ethyl acetate, benzene, toluene, xylene, dimethylformamide and dimethylacetamide. While the reaction temperature varies depending on the reagents and solvents to be used, it is generally from -20°C to the boiling point of the solvent.

The condensation reaction of the compound of the formula (II) and the compound of the formula (IV) is carried out in a suitable solvent. Examples of the solvent include organic solvents such as tetrahydrofuran, diethyl ether, diisopropyl ether, dioxane, dichloromethane, chloroform, carbon tetrachloride, ethyl acetate, benzene, toluene, xylene, dimethylformamide and dimethylacetamide. The reaction is carried out in the presence of, where necessary, a base or a condensing agent at a temperature of from -20°C to the boiling point of the solvent.

Examples of the base to be used as necessary include alkali metal hydroxides such as sodium hydroxide and potassium hydroxide, alkali metal carbonates such as sodium carbonate and potassium carbonate, alkali metal hydrogencarbonates such as sodium hydrogencarbonate and potassium hydrogencarbonate, alkali metal hydrides such as sodium hydride, and organic bases such as triethylamine, pyridine, picolin and N-methylmorpholine. Where necessary, an alkali metal hydroxide may be used in two phases of the above-mentioned organic solvent and water, using a phase transfer catalyst such as tetrabutylammonium bromide and benzyltriethylammonium iodide. The condensing agent is preferably one used for amide synthesis, and is exemplified by dicyclohexylcarbodiimide, N-ethyl-N'-(3-dimethylaminomethyl)carbodiimide hydrochloride, diphenylphosphoryl azide and N-methyl-2-chloropyridinium iodide.

A compound of the formula (II) wherein a is 0, which has the formula 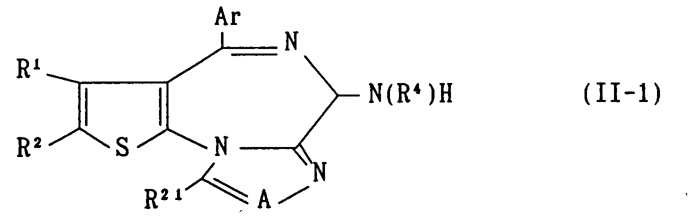
wherein each symbol is as defined above, can be obtained by reacting a compound of the formula 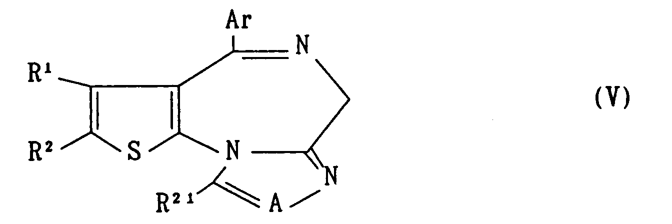
wherein each symbol is as defined above, with dialkyl carbonate (e.g. diethyl carbonate) in the presence of a base (e.g. sodium hydride, potassium tert-butoxide, lithium diisopropylamide and butyl lithium) to introduce alkoxycarbonyl (e.g. ethoxycarbonyl) into the 6-position; reacting the obtained compound with O-(2,4-dinitrophenyl)hydroxylamine to give a compound of the formula 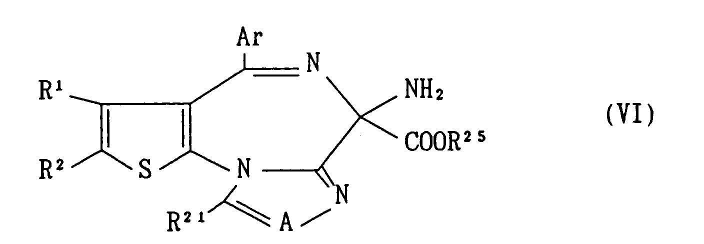
wherein R²⁵ is alkyl such as ethyl and other symbols are as defined above; subjecting the compound of the formula (VI) to hydrolysis in water or a mixed solvent of water and organic solvent (preferably, methanol, ethanol, diethyl ether, tetrahydrofuran and dioxane) in the presence of a base (e.g. sodium hydroxide, potassium hydroxide, barium hydroxide and lithium hydroxide) at a temperature of from about 0°C to the boiling point of the solvent used; converting the obtained reaction mixture to acidic using an acid such as hydrochloric acid, sulfuric acid, hydrobromic acid, trifluoroacetic acid and trifluoromethanesulfonic acid for decarboxylation to give a compound of the formula 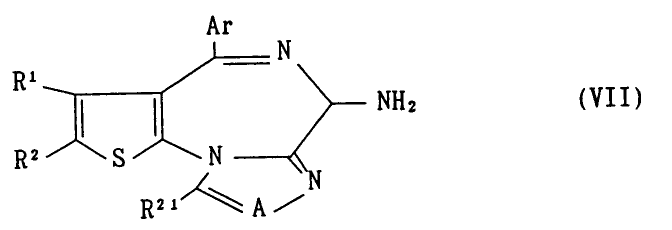
wherein each symbol is as defined above; and reacting the compound (VII) with an alkyl halide of the formula

R⁴Q (VIII)

wherein Q is halogen and R⁴ is as defined above, in a suitable solvent (e.g. diethyl ether, diisopropyl ether, tetrahydrofuran, dioxane, benzene, toluene, xylene and dimethylformamide) in the presence of a base (e.g. sodium hydride, potassium tert-butoxide, lithium diisopropylamide, butyl lithium, pyridine, triethylamine, potassium carbonate, sodium carbonate and sodium hydrogencarbonate) at a temperature of from -20°C to the boiling point of the solvent used, or reacting the compound (VII) with an aldehyde in the presence of a reducing agent such as sodium borohydride and sodium cyanoborohydride.

A compound of the formula (II) wherein a is 1 to 6 can be obtained by, after introducing alkoxycarbonyl into the 6-position in the same manner as above, reacting the resulting compound with a compound of the formula 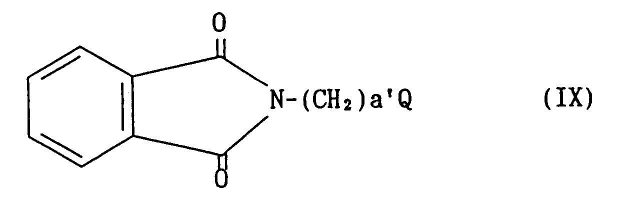
wherein a' is an integer of 1 to 6 and Q is halogen, to give a compound of the formula 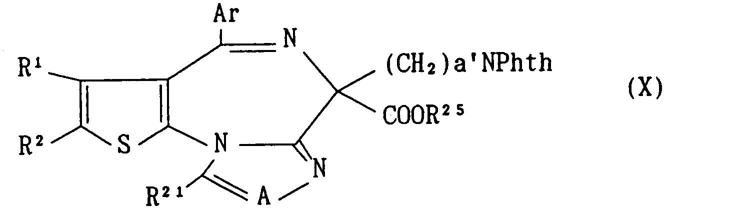
wherein Phth means phthaloyl and other symbols are as defined above; subjecting the compound (X) to hydrolysis in water or a mixed solvent of water and organic solvent (preferably, methanol, ethanol, diethyl ether, tetrahydrofuran and dioxane) in the presence of a base (e.g. sodium hydroxide, potassium hydroxide, barium hydroxide and lithium hydroxide) at a temperature of from about 0°C to the boiling point of the solvent used; converting the obtained reaction mixture to acidic using an acid such as hydrochloric acid, sulfuric acid, hydrobromic acid, trifluoroacetic acid, trifluoromethanesulfonic acid and the like for decarboxylation; subjecting the obtained compound to deprotection in a suitable solvent (e.g. water, methanol, ethanol, isopropyl alcohol, tetrahydrofuran, dioxane and mixed solvent thereof) by adding hydrazine at a temperature of from about 0°C to the boiling point of the solvent used to give a compound of the formula 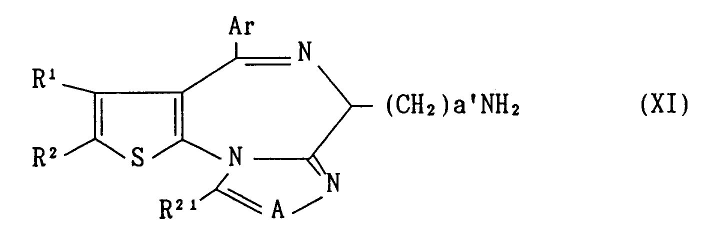
wherein each symbol is as defined above; and reacting the compound (XI) with an alkyl halide of the formula (VIII) in a suitable solvent (e.g. diethyl ether, diisopropyl ether, tetrahydrofuran, dioxane, benzene, toluene, xylene and dimethylformamide) in the presence of a base (e.g. sodium hydride, potassium tert-butoxide, lithium diisopropylamide, butyl lithium, pyridine, triethylamine, potassium carbonate, sodium carbonate and sodium hydrogencarbonate) at a temperature of from -20°C to the boiling point of the solvent used, or reacting the compound (XI) with an aldehyde in the presence of a reducing agent such as sodium borohydride and sodium cyanoborohydride.

A compound of the formula (V) wherein A is a nitrogen atom can be obtained by reacting a compound of the formula 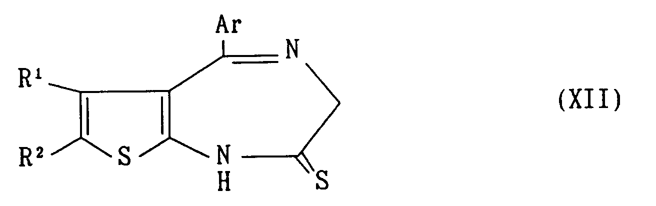
wherein each symbol is as defined above, which can be obtained by reference to Japanese Patent Unexamined Publication Nos. 79185/1989 and 156982/1989, with a compound of the formula

R²¹CONHNH₂ (XIII)

wherein R²¹ is as defined above, or by reacting a compound of the formula 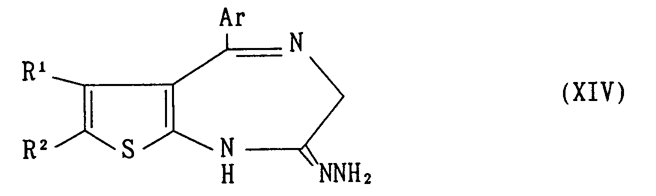
wherein each symbol is as defined above, which can be obtained by reacting a compound of the formula (XII) with a hydrazine hydrate, with a compound of the formula

R²¹COOH (XV)

wherein R²¹ is as defined above, or a reactive derivative thereof or a compound of the formula

R²¹C(OR²⁶)₃ (XVI)

wherein R²⁶ is alkyl such as methyl and ethyl and R²¹ is as defined above.

The reaction between the compound of the formula (XII) and the compound of the formula (XIII) is generally carried out in a solvent inert to the reaction (e.g. benzene, toluene, xylene, tetrahydrofuran, dioxane and mixed solvent thereof) in the presence of an organic acid (e.g. acetic acid and propionic acid), an inorganic acid (e.g. hydrochloric acid and sulfuric acid) or silica gel at a temperature of from room temperature to the refluxing temperature of the solvent used for 30 minutes to 5 hours. The reaction between the compound of the formula (XII) and hydrazine hydrate generally proceeds in a solvent inert to the reaction (e.g. methanol, ethanol, propanol, isopropyl alcohol and butanol) at 0-40°C for about 5 minutes to 3 hours.

The reaction between the compound of the formula (XIV) and the compound of the formula (XV), a reactive derivative thereof or the compound of the formula (XVI) is generally carried out in a solvent inert to the reaction (e.g. benzene, toluene, xylene, tetrahydrofuran, dioxane and mixed solvent thereof) in the presence of an organic acid (e.g. acetic acid and propionic acid), an inorganic acid (e.g. hydrochloric acid and sulfuric acid) or silica gel at a temperature of from room temperature to the refluxing temperature of the solvent used for 30 minutes to 6 hours.

A compound of the formula (V) wherein A is CH is obtained by reacting a compound of the formula (XII) with a compound of the formula 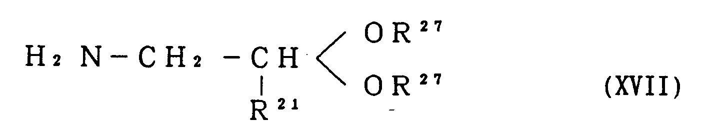
wherein R²⁷ is alkyl such as methyl and ethyl or aralkyl such as benzyl and R²¹ is as defined above.

The reaction is generally carried out in a solvent inert to the reaction (e.g. methanol, ethanol, dioxane, dimethylformamide, tetrahydrofuran, benzene, toluene, xylene and mixed solvent thereof) in the presence of an acid catalyst (e.g. mineral acids such as hydrochloric acid, sulfuric acid and polyphosphoric acid, lower fatty acids such as formic acid, acetic acid and propionic acid, and organic sulfonic acid such as methanesulfonic acid and p-toluenesulfonic acid) at room temperature to 150°C, preferably under reflux of the solvent. When the acid catalyst *per se* to be used in the above reaction is liquid, the catalyst may act as a solvent. When the reaction is carried out without solvent, it is carried out at a temperature somewhat higher than the melting point of the compound of the formula (V), generally at 150-220°C.

Of the compounds of the formula (I), compounds of the formula (Ib) can be produced as in the following.
Method 1 : A compound of the formula 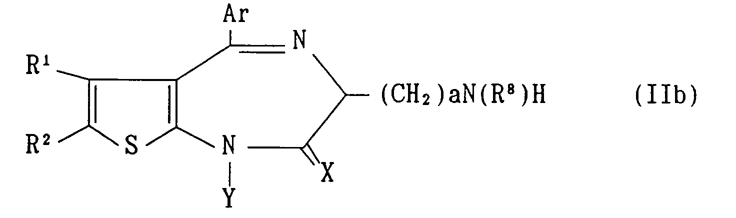
wherein each symbol is as defined above, is reacted with a sulfonic acid halide of the formula

Q-SO₂R⁹ (IIIb)

wherein Q is halogen such as chlorine and other symbols are as defined above, in an inert solvent such as dichloromethane, chloroform, benzene, toluene, carbon tetrachloride, tetrahydrofuran and dioxane in the presence of, where necessary, a base (e.g. triethylamine, pyridine and N-methylmorpholine) to give a compound of the formula 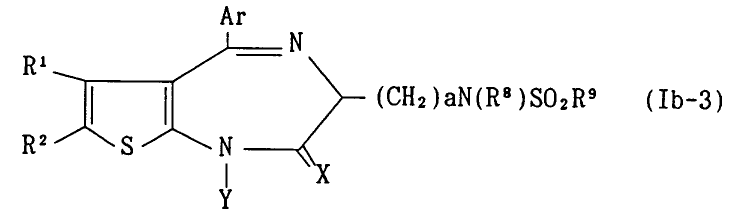
wherein each symbol is as defined above.
Method 2 : A compound of the formula (IIb) is reacted with a halide of the formula

Q-COOR¹⁰ (IVb)

wherein each symbol is as defined above, in an inert solvent such as chloroform, dichloromethane, benzene, toluene, dimethylformamide, tetrahydrofuran and dioxane, whereby a compound of the formula 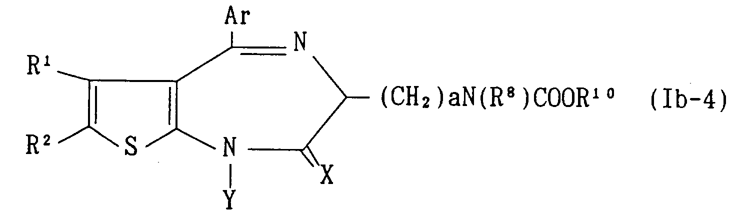
wherein each symbol is as defined above, is obtained.
Method 3 : A compound of the formula 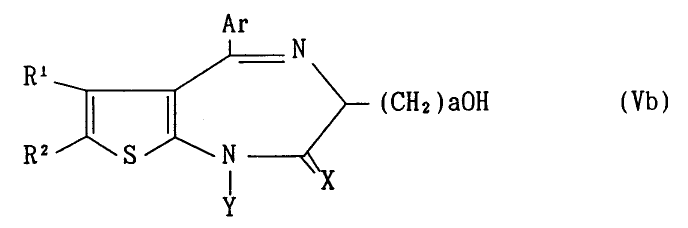
wherein each symbol is as defined above, is reacted with a halide of the formula

Q-CON(R¹¹)(R¹²) (VIb)

wherein each symbol is as defined above, or with an isocyanate of the formula

OCN-R²⁸ (VIIb)

wherein R²⁸ is either R¹¹ or R¹², in an inert solvent such as dichloromethane, chloroform, benzene, toluene, dimethylformamide and carbon tetrachloride, whereby a compound of the formula 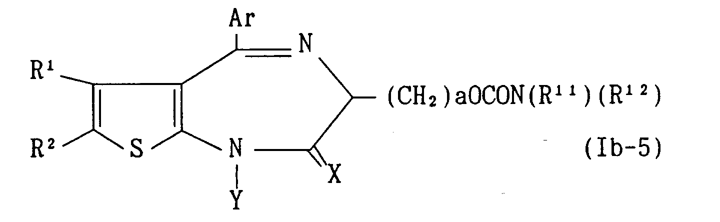
wherein each symbol is as defined above, is obtained.
Method 4 : A compound of the formula 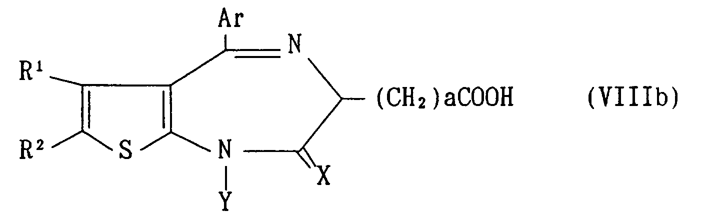
wherein each symbol is as defined above, is reacted with a compound of the formula

HN(R¹³)(R¹⁴) (IXb)

wherein each symbol is as defined above, whereby a compound of the formula 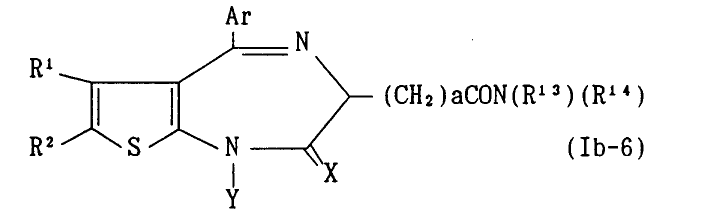
wherein each symbol is as defined above, is obtained.

The reaction is carried out in a suitable solvent (e.g. organic solvent such as tetrahydrofuran, diethyl ether, diisopropyl ether, dioxane, dichloromethane, chloroform, carbon tetrachloride, ethyl acetate, benzene, toluene, xylene, dimethylformamide and dimethylacetamide) in the presence of, where necessary, a base or a dehydratively condensing agent at about -20°C to the boiling point of the solvent. Examples of the base to be used as necessary include triethylamine, pyridine and N-methylmorpholine. The dehydratively condensing agent is preferably one conventionally used for peptide synthesis, which is exemplified by dicyclohexylcarbodiimide, N-ethyl-N'-(3-dimethylaminomethyl)carbodiimide hydrochloride, diphenylphosphoryl azide, N-methyl-2-chloropyridinium iodide and molecular sieve.
Method 5 : A compound of the formula (Vb) is reacted with an acid halide of the formula

Q-COR¹⁵ (Xb)

wherein each symbol is as defined above, or an acid anhydride of the formula

(R¹⁵CO)₂O (XIb)

wherein each symbol is as defined above, in an inert solvent such as dichloromethane, chloroform, benzene, toluene, dimethylformamide and carbon tetrachloride, whereby a compound of the formula 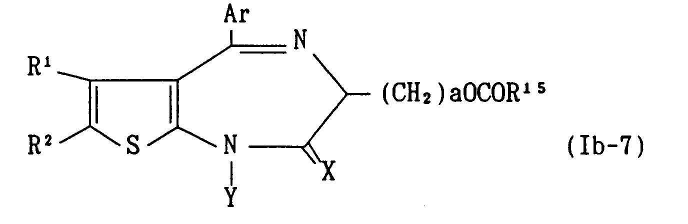
wherein each symbol is as defined above, is obtained.
Method 6 : A compound of the formula (Vb) is reacted with a compound of the formula

Q-SO₂R¹⁶ (XIIb)

wherein each symbol is as defined above, to give a compound of the formula 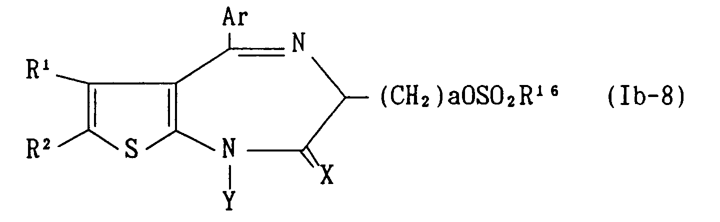
wherein each symbol is as defined above.
Method 7 : A compound of the formula 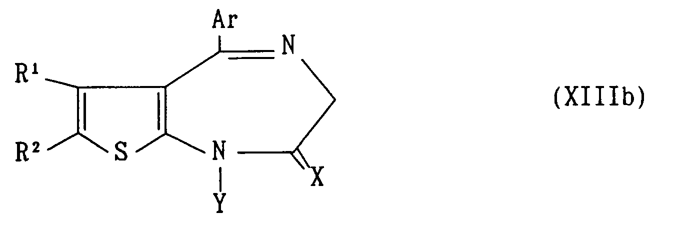
wherein each symbol is as defined above, is reacted with a dialkyl carbonate such as diethyl carbonate in the presence of a base such as sodium hydride, potassium tert-butoxide, lithium diisopropylamide and butyl lithium to introduce alkoxycarbonyl into the 3-position, and the obtained compound is reacted with a halide of the formula

Q-(CH₂)aCOR¹⁷ (XIVb)

wherein each symbol is as defined above, to give a compound of the formula 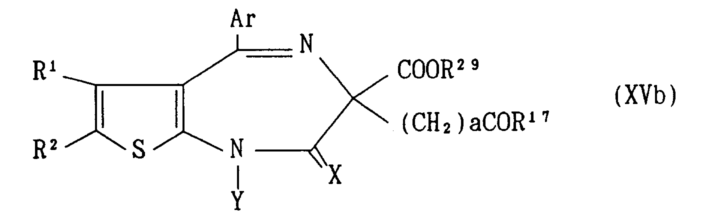
wherein R²⁹ is alkyl such as methyl and ethyl and other symbols are as defined above, which is then subjected to hydrolysis in water or a mixed solvent of water and suitable solvent (e.g. methanol, ethanol, tetrahydrofuran and dioxane) in the presence of a base such as sodium hydroxide, potassium hydroxide, sodium carbonate, potassium carbonate, sodium hydrogencarbonate and barium hydroxide at a temperature of from 0°C to the boiling point of the solvent used and decarboxylation using an acid (e.g. hydrochloric acid, sulfuric acid, hydrobromic acid, trifluoroacetic acid and trifluoromethanesulfonic acid), whereby a compound of the formula 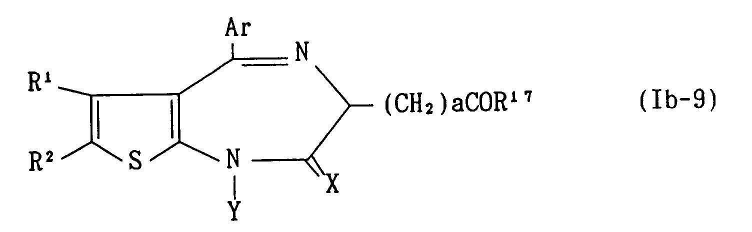
wherein each symbol is as defined above, is obtained.
Method 8 : A compound of the formula (Vb) is halogenated with a halogenating agent such as phosphorus oxychloride, phosphorous tribromide, chlorine, bromine and N-bromosuccinimide in an inert solvent such as dichloromethane, chloroform, carbon tetrachloride, benzene, toluene, dioxane and dimethylformamide and reacted with a thiol or a thiolate of the formula

R¹⁸SH or R¹⁸SNa (XVIb)

wherein R¹⁸ is as defined above, in a suitable solvent (e.g. methanol, ethanol, dimethylacetamide and dimethylformamide) to give a compound of the formula 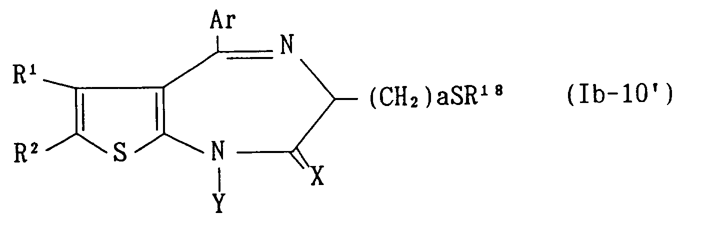
wherein each symbol is as defined above. This compound is oxidized using an oxidizing agent (e.g. hydrogen peroxide, potassium permanganate, sodium hypochlorite, ozone and ruthenium oxide) in ethanol, methanol, acetic acid or a mixed solvent of acetic acid and water, whereby a compound of the formula 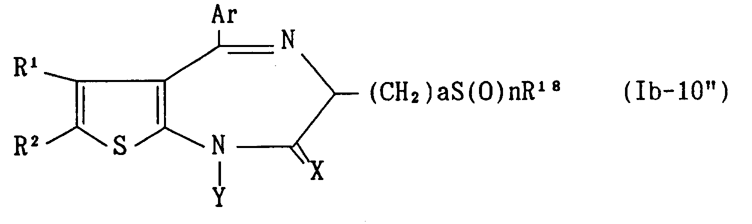
wherein n' is 1 or 2 and other symbols are as defined above, is obtained.

### Production method of intermediates:

A compound of the formula (IIb) wherein a is 0 is obtained by reacting a compound of the formula 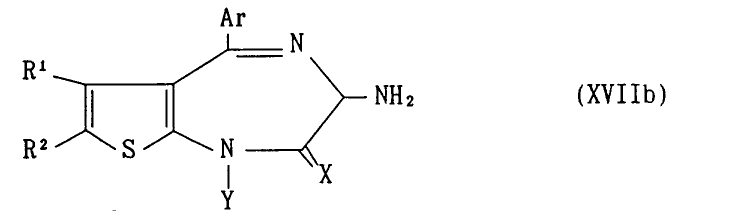
wherein each symbol is as defined above, which is obtained by reference to the method described in Japanese Patent Unexamined Publication No. 28181/1990, with an alkyl halide of the formula

R⁸'-Q (XVIIIb)

wherein R⁸' is R⁸ other than hydrogen and Q is as defined above, in a suitable solvent (e.g. diethyl ether, diisopropyl ether, tetrahydrofuran, dioxane, benzene, toluene, xylene and dimethylformamide) in the presence of a base (e.g. sodium hydride, potassium tert-butoxide, lithium diisopropylamide, butyl lithium, pyridine, triethylamine, potassium carbonate, sodium carbonate and sodium hydrogencarbonate) at about -20°C to the boiling point of the solvent used, or by reacting with aldehyde in the presence of a reducing agent such as sodium borohydride and sodium cyanoborohydride.

A compound of the formula (IIb) wherein a is 1-6 can be synthesized by the following synthetic route. 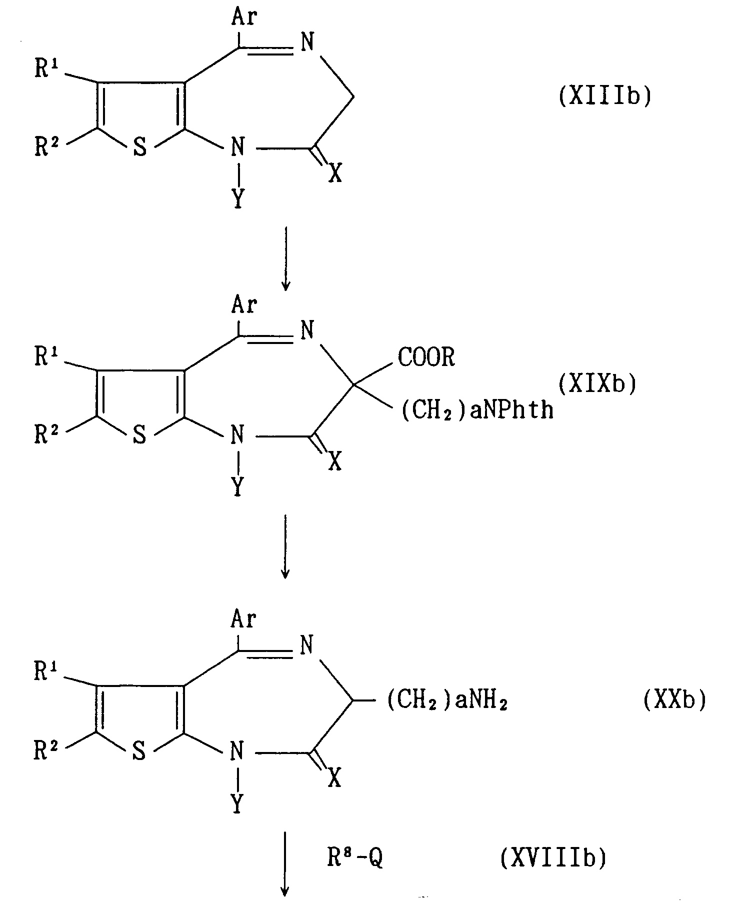 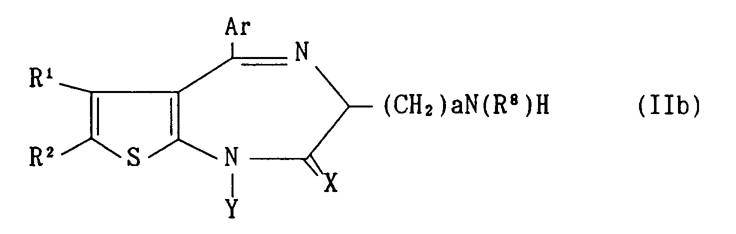
wherein R is alkyl such as methyl and ethyl, Phth is phthaloyl and other symbols are as defined above.

A compound of the formula (XIIIb) is reacted with dialkyl carbonate (e.g. diethyl carbonate) in the presence of a base (e.g. sodium hydride, potassium tert-butoxide, lithium diisopropylamide and butyl lithium) to introduce alkoxycarbonyl (e.g. ethoxycarbonyl) into the 3-position, and reacted with a halide of the formula

Q(CH₂)aNPhth

wherein Phth is phthaloyl and other symbols are as defined above, to give a compound of the formula (XIXb). This compound of the formula (XIXb) is subjected to hydrolysis in water or a mixed solvent of water and organic solvent (e.g. methanol, ethanol, diethyl ether, tetrahydrofuran and dioxane) in the presence of a base (e.g. sodium hydroxide, potassium hydroxide, barium hydroxide and lithium hydroxide) at a temperature of from about 0°C to the boiling point of the solvent used, and decarboxylation by converting the mixture to acidic using an acid such as hydrochloric acid, sulfuric acid, hydrobromic acid, trifluoroacetic acid and trifluoromethanesulfonic acid. Deprotection by adding hydrazine in a suitable solvent (e.g. water, methanol, ethanol, isopropyl alcohol, tetrahydrofuran, dioxane and mixed solvent thereof) at a temperature of from 0°C to the boiling point of the solvent used gives a compound of the formula (XXb). The compound of the formula (XXb) is reacted with an alkyl halide of the formula (XVIIIb) in a suitable solvent (e.g. diethyl ether, diisopropyl ether, tetrahydrofuran, dioxane, benzene, toluene, xylene and dimethylformamide) in the presence of a base (e.g. sodium hydride, potassium tert-butoxide, lithium diisopropylamide, butyl lithium, pyridine, triethylamine, potassium carbonate, sodium carbonate and sodium hydrogencarbonate) at a temperature of from -20°C to the boiling point of the solvent used, or reacted with aldehyde in the presence of a reducing agent such as sodium borohydride and sodium cyanoborohydride, whereby a compound of the formula (IIb) is obtained.

A compound of the formula (Vb) wherein a is 0 can be synthesized by the following synthetic route. 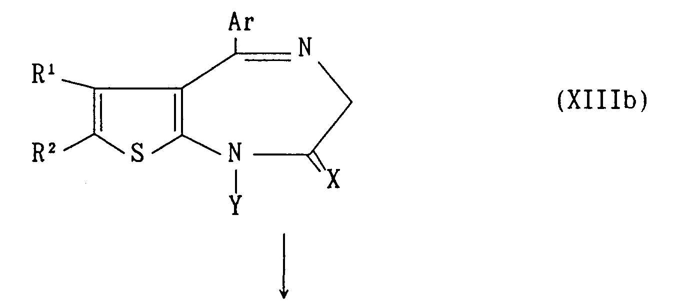 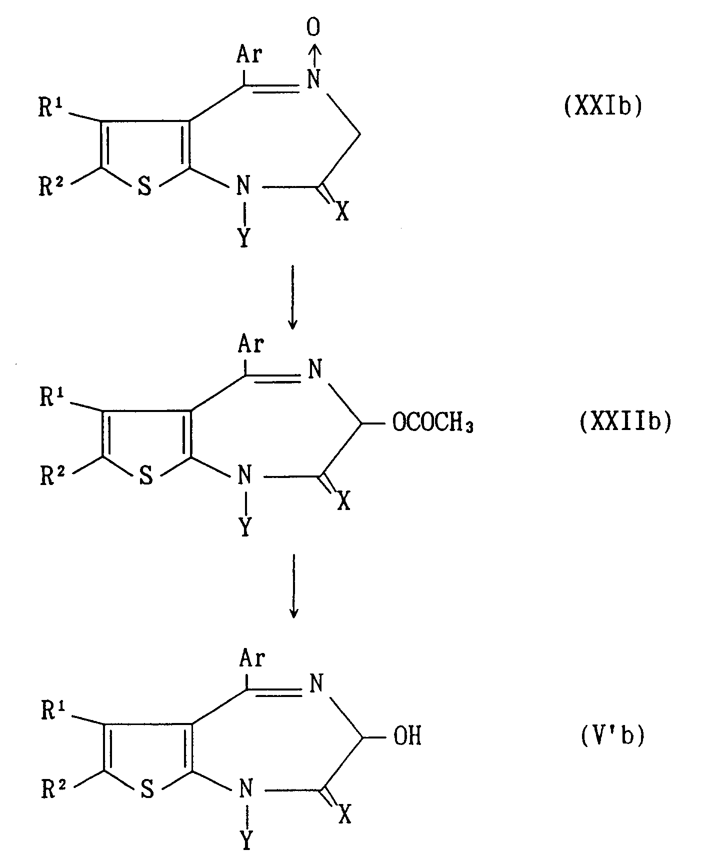
wherein each symbol is as defined above.

A compound of the formula (XIIIb) is converted to N-oxide (XXIb) by the use of a peracid such as m-chloroperbenzoic acid, hydrogen peroxide and peracetic acid in an inert solvent such as chloroform, carbon tetrachloride, dichloroethane, benzene and toluene, and subjected to Polonovski rearrangement in acetic anhydride to give a compound of the formula (XXIIb), which is then hydrolyzed by the reaction with a base, such as sodium hydroxide, barium hydroxide and potassium hydroxide, in a mixed solvent of water and suitable organic solvent (e.g. methanol, ethanol and isopropanol), whereby a compound of the formula (V'b) is obtained.

A compound of the formula (Vb) wherein a is 1 to 6 can be obtained in the same manner as above, by introducing alkoxycarbonyl into the 3-position of the compound of the formula (XIIIb), reacting the obtained compound with a halide of the formula

Q-(CH₂)aOCOR' (XXIIIb)

wherein R' is an alkyl such as methyl and ethyl and other symbols are as defined above, and subjecting the obtained compound of the formula 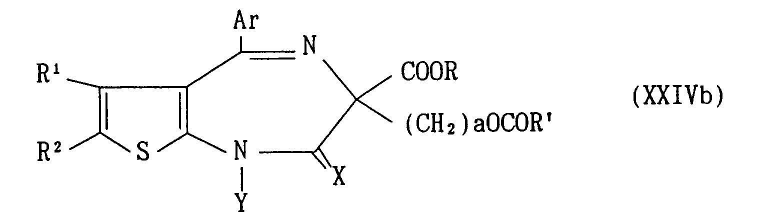
wherein each symbol is as defined above, to decarboxylation and hydrolysis in the same manner as above.

Compounds of the formula (IIb) and (Vb) wherein a is 1 to 6 are also directly obtained by reacting a compound of the formula (XIIIb) with a compound of the formula

Q-(CH₂)aNPhth or

Q-(CH₂)aOCOR'

wherein each symbol is as defined above, in an inert solvent such as tetrahydrofuran, dioxane, diethyl ether, benzene, toluene and dimethylformamide in the presence of a base (e.g. sodium hydroxide, potassium tert-butoxide, lithium diisopropylamide and butyl lithium). The reaction generally proceeds at a temperature of from -50°C to 0°C.
The obtained compounds are respectively subjected to decarboxylation and hydrolysis to give compounds of the formulas (IIb) and (Vb).

A compound of the formula (VIIIb) can be synthesized by the following synthetic route. 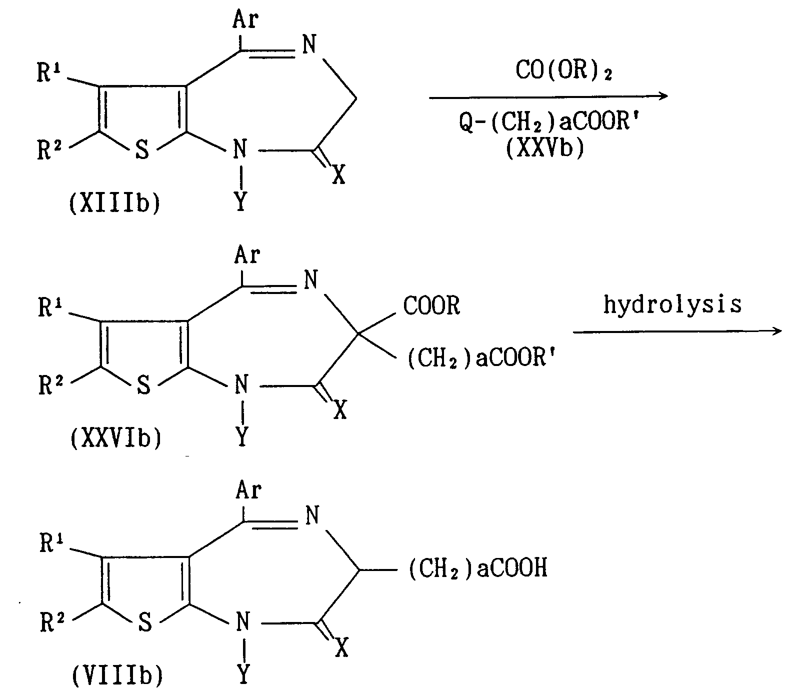
wherein each symbol is as defined above.

A compound of the formula (XIIIb) is reacted with dialkyl carbonate such as diethyl carbonate in the presence of a base such as sodium hydroxide, potassium tert-butoxide, lithium diisopropylamide and butyl lithium to introduce alkoxycarbonyl such as ethoxycarbonyl into the 3-position of the diazepine ring, and reacted with a haloester of the formula

Q-(CH₂)aCOOR'

wherein R', Q and a are as defined above, to give a compound of the formula (XXVIb). The compound of the formula (XXVIb) is subjected to hydrolysis in water or a mixed solvent of water and organic solvent (e.g. methanol, ethanol, tetrahydrofuran and dioxane) in the presence of a base, such as sodium hydroxide, potassium hydroxide, sodium carbonate, potassium carbonate, sodium hydrogencarbonate and barium hydroxide, at a temperature of from 0°C to the boiling point of the solvent used, and decarboxylation by converting the mixture to acidic using an acid such as hydrochloric acid, sulfuric acid, hydrobromic acid, trifluoroacetic acid and trifluoromethanesulfonic acid, whereby a compound of the formula (VIIIb) is obtained.

The compounds of the formula (I) thus obtained are separated and purified from reaction mixtures by a method known *per se*, such as recrystallization and column chromatography.

The compounds of the formula (I) can be converted to salts by treating with inorganic acid (e.g. hydrochloric acid, hydrobromic acid, sulfuric acid, phosphoric acid and nitric acid), organic acid (e.g. acetic acid, propionic acid, succinic acid, glycolic acid, lactic acid, malic acid, tartaric acid, citric acid, maleic acid, fumaric acid, methanesulfonic acid, benzenesulfonic acid, p-toluenesulfonic acid and ascorbic acid), inorganic base (e.g. sodium hydroxide, potassium hydroxide, calcium hydroxide, magnesium hydroxide, zinc hydroxide and ammonium hydroxide), organic base (e.g. methylamine, diethylamine, triethylamine, dicyclohexylamine, triethanolamine, ethylenediamine, trishydroxymethylaminomethane, quinine, guanidine and cinchonine) or amino acid (e.g. lysine, ornithine, arginine and alanine) by a conventional method. The present invention also encompasses hydrates and other solvates.

When the compound of the present invention has a chiral carbon atom, it is generally obtained as a racemate. The racemate can be resolved into optical isomers by a conventional method. Such optical isomers can be also produced by using an optically active starting material. Respective diastereomers can be purified by fractional crystallization or chromatography.

The compounds of the present invention and salts thereof have superior antagonistic action on cholecystokinin and gastrin, as well as strong and long-lasting suppressive action on the secretion of pancreatic enzymes and gastric acid. Accordingly, they are useful as drugs acting on the central and peripheral nerves and drugs for the prevention and treatment of pancreatic disorders and gastrointestinal ulcers. In addition, the compounds of the present invention are expected to show anxiolytic action based on the anxiolytic action on cholecystokinin, and are useful as anxiolytic drugs. The experiment methods for the pharmacological action of the compounds of the present invention are shown in the following.

### EXPERIMENTAL EXAMPLE 1:

### Cholecystokinin (CCK-B) receptor binding test

Rat cerebral cortex was homogenized (Polytron, 15,000 rpm, 60 seconds) in 20-fold amount of ice-cooled 0.32 M sucrose solution and centrifuged at 1,000 g for 10 minutes. The supernatant was further centrifuged at 40,000 g for 15 minutes. For washing, the obtained pellets were homogenized (Polytron, 15,000 rpm, 10 seconds)-centrifuged (40,000 g, 10 minutes) twice in 10 mM HEPES buffer solution (pH 7.4, containing 130 mM NaCl and 5 mM MgCl₂). The pellets finally obtained were homogenized (Polytron, 15,000 rpm, 20 seconds) in 30-fold amount of buffer for assay (5 mM HEPES, pH 6.5, containing 130 mM NaCl, 5 mM MgCl₂, 0.02% bacitracin, 1 mg/ℓ phenylmethanesulfonyl fluoride) to prepare a synapse membrane. ³H-CCK₈ (50 µl, final concentration 0.5 nM) and a test solution or a solvent (50 µl) were added to the synapse membrane (0.9 ml) and reacted at 22°C for 40 minutes. After filtration by suction through glass fiber filter (Whatman GF/B), the filter was immediately washed 3 times with ice-cooled 10 mM HEPES buffer (pH 7.4, 3 ml) and the radioactivity on the filter was assayed. The binding amount in the presence of 1 µM CCK₈ was considered to reflect nonspecific binding.

### EXPERIMENTAL EXAMPLE 2:

### Cholecystokinin (CCK-A) receptor binding test

Rat pancreas was homogenized (Polytron, 15,000 rpm, 60 seconds) in 20-fold amount of ice-cooled 10 mM HEPES buffer solution (pH 7.4, containing 130 mM NaCl and 5 mM MgCl₂) and centrifuged at 40,000 g for 15 minutes. For washing, the obtained pellets were homogenized (Polytron, 15,000 rpm, 10 seconds)-centrifuged (40,000 g, 10 minutes) twice in 10 mM HEPES buffer. The pellets finally obtained were homogenized (Polytron, 15,000 rpm, 20 seconds) in 60-fold amount of buffer for assay (10 mM HEPES, pH 7.4, containing 130 mM NaCl, 5 mM MgCl₂, 0.02% bacitracin, 1 mg/ℓ phenylmethanesulfonyl fluoride) to prepare a synapse membrane. ³H-CCK₈ (50 µl, final concentration 0.5 nM) and a test solution or a solvent (50 µl) were added to the synapse membrane (0.9 ml) and reacted at 22°C for 60 minutes. After filtration by suction through glass fiber filter (Whatmann GF/B), the filter was immediately washed 3 times with ice-cooled 10 mM HEPES buffer (pH 7.4, 3 ml) and the radioactivity on the filter was assayed. The binding amount in the presence of 1 µM CCK₈ was considered to reflect nonspecific binding.

The effects of the test compound on binding with CCK-receptor as tested in EXPERIMENTAL EXAMPLEs 1 and 2 were evaluated by calculating the inhibitory ratio by the following formula and determining the concentration (IC₅₀) necessary for inhibiting the specific binding by 50%. The results of EXPERIMENTAL EXAMPLE 1 are shown in the following table.

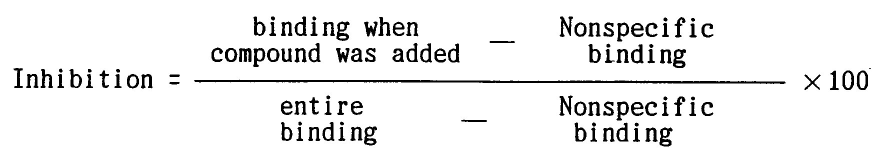

| Test compound | CCK-B receptor binding IC₅₀ (nM) |
|---|---|
| Compound of Example 1 | 26 |
| Compound of Example 2 | 26 |
| Compound of Example 3 | 51 |
| Compound of Example 4 | 33 |
| Compound of No. 12 | 62 |
| Compound of No. 17 | 47 |
| Compound of No. 20 | 43 |
| Compound of No. 22 | 10 |
| Compound of No. 82 | 65 |
| (+) compound of Example 401 | 9.4 |

The compounds of the formula (I) of the present invention suppress expression of CD18 on leukocytes, thereby inhibiting adhesion of leukocyte to vascular endothelial cells and leukocyte infiltration *in vivo*. In view of such actions, the compounds of the present invention are expected to be useful as drugs for the treatment and prevention of various inflammatory diseases and allergic diseases, where the cell adhesion is concerned with the onset and progress, and usable for the prevention and treatment of autoimmune diseases and rejection in organ transplantation, and further, for the prevention of metastasis of malignant cells. In the following, pharmacological test methods are shown.

### EXPERIMENTAL EXAMPLE 3:

### Inhibitory effect on adhesion of U937 cells (human histiocytic leukemia cells) to human umbilical vein-derived endothelial cells (HUVECs)

HUVECs were suspended in 199 medium supplemented with fetal calf serum (20%), endothelial cell growth factor derived from bovine brain (20 µg/ml) and heparin (100 µg/ml). Cells were plated into 96-well culture plates coated with collagen and cultured at 37°C in the presence of 5% CO₂. When cells were grown to confluence, interleukin-1 (10 U/ml) was added to the cells and the plates were incubated for additional 24 hours. After the plates were washed, leukotrien B₄ (1 µM), a test compound and U937 cells (3×10⁵ cells/well) were added to the wells, then the plates were incubated for 30 minutes. After non-adherent cells were removed by inversion of the plates, Rose bengal solution (0.25% in phosphate-buffered saline) was added to the cells. After being left for 5 minutes, the plates were washed twice with 199 medium to remove non-adherent cells. Rose bengal dye incorporated into cells was extracted by adding 50% ethanol in phosphate-buffered saline and leaving the plates for 20 minutes. Absorbance at a wavelength of 570 nm was measured with a 96-well plate reader and the value obtained by subtracting the absorbance of wells of HUVECs alone was defined as adhesion of U937 cells.

### EXPERIMENTAL EXAMPLE 4:

### Effect on CD18 expression on human peripheral blood neutrophils

Effect on expression of CD18 molecule was examined; CD18 molecule is composing a β chain of LFA-1 (lymphocyte function associated antigen-1) which is one of adhesion molecules expressing on the surface of almost all leukocytic cells. Human peripheral blood neutrophils prepared with dextran were suspended in RPMI1640 medium containing fetal calf serum (20%) and added into 96-well filtration plates (2×10⁵ cells/well) with leukotrien B₄ (1 µM) and a test compound. After incubated for 90 minutes at 37°C in the presence of 5% CO₂, cells were washed once with RPMI1640 medium and added with mouse anti-human CD18 monoclonal antibody (4 µg/ml) and left standing for 1 hour on ice. After washed once, cells were added with peroxidase-conjugated anti-mouse immunoglobulin antibody (2.5 µg/ml) and left standing for 1 hour on ice. After cells were washed twice, substrate for peroxidase (2,2'-azino-bis(3-ethylbenzothiazoline-6-sulfonic acid)) was added to the cells and the plates were left standing for 30 minutes at room temperature. Absorbance at a wavelength of 405 nm was measured with a 96-well plate reader and taken as an indicator of CD18 antigen expression.

### EXPERIMENTAL EXAMPLE 5:

### Effect on oxazolone-induced ear edema in mice

Mice were sensitized by applying 50 µl of oxazolone solution (50 mg/ml in acetone) to their shaved abdomen. On the 6th day after the sensitization, 5 µl of oxazolone solution was applied to each of the inside and the outside of the right ear. After 24 hours, the right and left ears were cut off with a puncher (6 mm in diameter) and weighed with an electric reading balance. The difference in weight between the treated (right) and untreated (left) ears was taken to reflect the extent of ear edema. A test compound was suspended in 0.5% methylcellulose solution and administered orally (0.1 ml/10 g body weight) on the 3rd, 4th, 5th and 6th days after the sensitization.

Moreover, acute toxicity of the compound of the present invention was examined using 6 male mice. A test compound was orally administered and the mice were monitored for 5 days. As a result, no death case was found in the dose of 1,000 mg/kg.

### [Industrial Applicability]

When the compound of the present invention or a pharmaceutically acceptable salt thereof is used as a pharmaceutical, pharmaceutically acceptable additives such as carriers, excipients, diluents and solubilizers (e.g. lactose, corn starch, talc, kaolin, physiological saline and sterilized water) are generally admixed therewith and formulated into the dosage form of, for example, tablet (sugar-coated tablet and film-coated tablet inclusive), capsule, powder, injection, transfusion, suppository or cataplasm, which can be administered safely to patients. While the dose varies depending on sex, age, body weight and symptom of the patients, it is preferably about 1 to 500 mg for an adult per day by oral administration.

### [Best Mode for Embodying the Invention]

The present invention is explained in more detail in the following by reference to Examples. Needless to say, the present invention is not limited to these examples. In the Examples, Reference Examples and Formulation Examples, "part" and "%" respectively mean "part by weight" and "% by weight", unless otherwise specified.

### Starting Material Preparation Example 1

5-(2-Chlorophenyl)-7-ethyl-1,3-dihydro-2H-thieno[2,3-e]1,4-diazepin-2-one (40 g) was dissolved in chloroform (600 ml) and phosphorus pentasulfide (117 g) was added with stirring. The mixture was refluxed for 3 hours. After the completion of the reaction, the reaction mixture was neutralized with a saturated aqueous solution of sodium bicarbonate, washed with water and dried over anhydrous magnesium sulfate. The solvent was distilled away under reduced pressure, and precipitated crystals were filtrated with diisopropyl ether and recrystallized from ethanol-chloroform to give 42 g of 5-(2-chlorophenyl)-7-ethyl-1,3-dihydro-2H-thieno[2,3-e]-1,4-diazepin-2-thione, melting point 198-199°C. The thione (42 g) obtained was suspended in methanol (300 ml), and 100% hydrazine·hydrate (19 ml) was added thereto under cooling. The mixture was stirred at room temperature for 2 hours. After the completion of the reaction, precipitated crystals were collected by filtration. Recrystallization from ethanoldimethylformamide gave 5-(2-chlorophenyl)-7-ethyl-1,3-dihydro-2H-thieno[2,3-e]-1,4-diazepine-2-hydrazone (34 g), melting point 214-216°C. Hydrazone (20 g) was dissolved in chloroform (200 ml), and cyclohexylcarbonyl chloride (10 g) was added with stirring. The mixture was stirred at room temperature for 1 hour. After the completion of the reaction, the reaction mixture was washed with a saturated aqueous solution of sodium bicarbonate and water, and dried over anhydrous magnesium sulfate. The resulting mixture was concentrated under reduced pressure, and the residue obtained was dissolved in toluene (200 ml). Acetic acid (5.4 ml) was added, and the mixture was refluxed for 3 hours. After the completion of the reaction, the reaction mixture was washed with a saturated aqueous solution of sodium bicarbonate and water, and dried over anhydrous magnesium sulfate. The resulting mixture was concentrated under reduced pressure, and the residue was subjected to silica gel column chromatography. The object fraction was concentrated under reduced pressure to give 15 g of 4-(2-chlorophenyl)-9-cyclohexyl-2-ethyl-6H-thieno[3,2-f][1,2,4]triazolo[4,3-a][1,4]diazepine, melting point 116-119°C.

### Starting Material Preparation Example 2

4-(2-Chlorophenyl)-9-cyclohexyl-2-ethyl-6H-thieno[3,2-f][1,2,4]triazolo[4,3-a][1,4]diazepine (3.4 g) and sodium hydride (0.56 g) were added to diethyl carbonate (50 ml), and the mixture was heated. After refluxing for 1 hour, the mixture was cooled to 20°C. O-(2,4-Dinitrophenyl)hydroxylamine (2.1 g) was added, and the mixture was stirred for 2 hours. After the completion of the reaction, the reaction mixture was poured on ice water, and the diethyl carbonate layer was separated. After washing twice with water, the organic layer was dried over anhydrous magnesium sulfate, and diethyl carbonate was distilled away under reduced pressure. Diisopropyl ether was added to the residue obtained, and the precipitated crystals were collected by filtration. Recrystallization from ethyl acetate gave ethyl (6-amino-4-(2-chlorophenyl)-9-cyclohexyl-2-ethyl-6H-thieno[3,2-f][1,2,4]triazolo[4,3-a][1,4]diazepin-6-yl)carboxylate (2.1 g), melting point 140-145°C. Ethyl (6-amino-4-(2-chlorophenyl)-9-cyclohexyl-2-ethyl-6H-thieno[3,2-f][1,2,4]triazolo[4,3-a][1,4]diazepin-6-yl)carboxylate (1.8 g) was dissolved in a mixture of ethanol (60 ml) and water (20 ml), and barium hydroxide·8 hydrate (1.14 g) was added. The mixture was stirred at room temperature for 24 hours, and the solvent was distilled away under reduced pressure. Water (50 ml) was added, and the mixture was adjusted to pH 2 with 1N hydrochloric acid. After stirring for 1 hour, the mixture was neutralized with an aqueous solution of sodium hydrogencarbonate, and extracted twice with chloroform. The organic layer was dried over anhydrous magnesium sulfate and the solvent was distilled away under reduced pressure. The resulting mixture was subjected to silica gel column chromatography. The solvent was distilled away, and the resulting crystals were recrystallized from diisopropyl ether to give 1.0 g of 6-amino-4-(2-chlorophenyl)-9-cyclohexyl-2-ethyl-6H-thieno[3,2-f][1,2,4]triazolo[4,3-a][1,4]diazepine, melting point 175-176°C.

### Starting Material Preparation Example 3

In the same manner as in Starting Material Preparation Example 2, 6-amino-4-(2-chlorophenyl)-2-ethyl-9-phenyl-6H-thieno[3,2-f][1,2,4]triazolo[4,3-a][1,4]diazepine was obtained from 4-(2-chlorophenyl)-2-ethyl-9-phenyl-6H-thieno[3,2-f][1,2,4]triazolo[4,3-a][1,4]diazepine.

### Starting Material Preparation Example 4

In the same manner as in Starting Material Preparation Example 2, 6-amino-4-(2-chlorophenyl)-9-cyclopentyl-2-ethyl-6H-thieno[3,2-f][1,2,4]triazolo[4,3-a][1,4]diazepine was obtained from 4-(2-chlorophenyl)-9-cyclopentyl-2-ethyl-6H-thieno[3,2-f][1,2,4]triazolo[4,3-a][1,4]diazepine.

### Starting Material Preparation Example 5

In the same manner as in Starting Material Preparation Example 2, 6-amino-4-(2-chlorophenyl)-9-cyclopropyl-2-ethyl-6H-thieno[3,2-f][1,2,4]triazolo[4,3-a][1,4]diazepine was obtained from 4-(2-chlorophenyl)-9-cyclopropyl-2-ethyl-6H-thieno[3,2-f][1,2,4]triazolo[4,3-a][1,4]diazepine.

### Starting Material Preparation Example 6

4-(2-Chlorophenyl)-9-cyclohexyl-2-ethyl-6H-thieno[3,2-f][1,2,4]triazolo[4,3-a][1,4]diazepine (3.4 g) and sodium hydride (0.56 g) were added to diethyl carbonate (50 ml), and the mixture was heated. After refluxing for 1 hour, the mixture was allowed to cool to room temperature, and 3-bromo-N-phthaloylpropylamine (2.3 g) was added. The mixture was refluxed for 1 hour, and the reaction mixture was poured on ice water. The diethyl carbonate layer was separated, and washed twice with water and dried over anhydrous magnesium sulfate. Diethyl carbonate was distilled away under reduced pressure, and the resulting mixture was subjected to silica gel column chromatography to give 2.4 g of ethyl 4-(2-chlorophenyl)-2-ethyl-9-cyclohexyl-6-(N-phthalylpropylamino)-6H-thieno[3,2-f][1,2,4]triazolo[4,3-a][1,4]diazepin-6-yl)carboxylate. Ethyl 4-(2-chlorophenyl)-9-cyclohexyl-2-ethyl-6-(N-phthalylpropylamino)-6H-thieno[3,2-f][1,2,4]triazolo[4,3-a][1,4]diazepin-6-yl)carboxylate (2.4 g) was dissloved in a mixture of ethanol (60 ml) and water (20 ml), and barium hydroxide·8 hydrate (1.14 g) was added. The mixture was stirred at room temperature for 24 hours, and the solvent was distilled away under reduced pressure. Water (50 ml) was added, and the mixture was adjusted to pH 2 with 1N hydrochloric acid. After stirring for 1 hour, the mixture was neutralized with an aqueous solution of sodium hydrogencarbonate and extracted twice with chloroform. The organic layer was dried over anhydrous magnesium sulfate and the solvent was distilled away under reduced pressure. The residue was dissloved in ethanol (50 ml) and hydrazine hydrate (1.25 g) was added. The mixture was refluxed for 3 hours. After the reaction, ethanol was distilled away under reduced pressure, and chloroform and water were added to the residue. After filtraion of the precipitated crystals, the chloroform layer was washed with a saturated aqueous solution of sodium bicarbonate. The separated chloroform layer was dried over anhydrous magnesium sulfate and the solvent was distilled away under reduced pressure. The resulting mixture was subjected to silica gel column chromatography to give 1.2 g of 6-(3-aminopropyl)-4-(2-chlorophenyl)-9-cyclohexyl-2-ethyl-6H-thieno[3,2-f][1,2,4]triazolo[4,3-a][1,4]diazepine.

The compounds obtained in the same manner as in Preparation Examples 2 and 6 are all novel compounds.

### Example 1

6-Amino-4-(2-chlorophenyl)-9-cyclohexyl-2-ethyl-6H-thieno[3,2-f][1,2,4]triazolo[4,3-a][1,4]diazepine (0.6 g) was dissolved in chloroform (15 ml), and 4-methoxyphenyl isocyanate (0.2 ml) was added. The mixture was stirred for 30 minutes. The reaction mixture was subjected to silica gel column chromatography, and the obtained crystals were recrystallized from methanol to give 0.49 g of N-(4-(2-chlorophenyl)-9-cyclohexyl-2-ethyl-6H-thieno[3,2-f][1,2,4]triazolo[4,3-a][1,4]diazepin-6-yl)-N'-(4-methoxyphenyl)urea, melting point 268-269°C.

### Example 2

In the same manner as in Example 1, N-(2-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea, melting point 260°C, was obtained from 6-amino-4-(2-chlorophenyl)-9-cyclohexyl-2-ethyl-6H-thieno[3,2-f][1,2,4]triazolo[4,3-a][1,4]diazepine and (2-chlorophenyl)isocyanate.

### Example 3

In the same manner as in Example 1, N-(4-(2-chlorophenyl)-9-cyclohexyl-2-ethyl-6H-thieno[3,2-f][1,2,4]triazolo[4,3- a][1,4]diazepin-6-yl)-N'-(3-methylphenyl)urea, melting point 259-260°C, was obtained from 6-amino-4-(2-chlorophenyl)-9-cyclohexyl-2-ethyl-6H-thieno[3,2-f][1,2,4]triazolo[4,3-a][1,4]diazepine and (3-methylphenyl)isocyanate.

### Example 4

In the same manner as in Example 1, N-(4-(2-chlorophenyl)-9-cyclohexyl-2-ethyl-6H-thieno[3,2-f][1,2,4]triazolo[4,3-a][1,4]diazepin-6-yl)-N'-(2-methylphenyl)urea, melting point 265-266°C, was obtained from 6-amino-4-(2-chlorophenyl)-9-cyclohexyl-2-ethyl-6H-thieno[3,2-f][1,2,4]triazolo[4,3-a][1,4]diazepine and (2-methylphenyl)isocyanate.

### Example 5

In the same manner as in Example 1, N-(4-(2-chlorophenyl)-9-cyclopentyl-2-ethyl-6H-thieno[3,2-f][1,2,4]triazolo[4,3-a][1,4]diazepin-6-yl)-N'-(3-methylphenyl)urea, melting point 246-247°C, was obtained from 6-amino-4-(2-chlorophenyl)-9-cyclopentyl-2-ethyl-6H-thieno[3,2-f][1,2,4]triazolo[4,3-a][1,4]diazepine and (3-methylphenyl)isocyanate.

### Example 6

In the same manner as in Example 1, N-(4-(2-chlorophenyl)-9-cyclopentyl-2-ethyl-6H-thieno[3,2-f][1,2,4]triazolo[4,3-a][1,4]diazepin-6-yl)-N'-(4-methoxyphenyl)urea, melting point 250-254°C, was obtained from 6-amino-4-(2-chlorophenyl)-9-cyclopentyl-2-ethyl-6H-thieno[3,2-f][1,2,4]triazolo[4,3-a][1,4]diazepine and (4-methoxyphenyl)isocyanate.

### Example 7

In the same manner as in Example 1, N-(4-(2-chlorophenyl)-9-cyclopropyl-2-ethyl-6H-thieno[3,2-f][1,2,4]triazolo[4,3-a][1,4]diazepin-6-yl)-N'-(4-methoxyphenyl)urea, melting point 249-251°C, was obtained from 6-amino-4-(2-chlorophenyl)-9-cyclopropyl-2-ethyl-6H-thieno[3,2-f][1,2,4]triazolo[4,3-a][1,4]diazepine and (4-methoxyphenyl)isocyanate.

### Example 8

In the same manner as in Example 1, N-(4-(2-chlorophenyl)-9-cyclopropyl-2-ethyl-6H-thieno[3,2-f][1,2,4]triazolo[4,3-a][1,4]diazepin-6-yl)-N'-(3-methylphenyl)urea, melting point, 270-272°C was obtained from 6-amino-4-(2-chlorophenyl)-9-cyclopropyl-2-ethyl-6H-thieno[3,2-f][1,2,4]triazolo[4,3-a][1,4]diazepine and (3-methylphenyl)isocyanate.

The compounds shown in the following Tables are produced in the same manner as in the above-mentioned Examples. In the Tables, Me means methyl, Et means ethyl, Bu means butyl, i-Bu means isobutyl, Ph means phenyl, c-C₃H₅ means cyclopropyl, c-C₅H₉ means cyclopentyl and c-C₆H₁₁ means cyclohexyl.

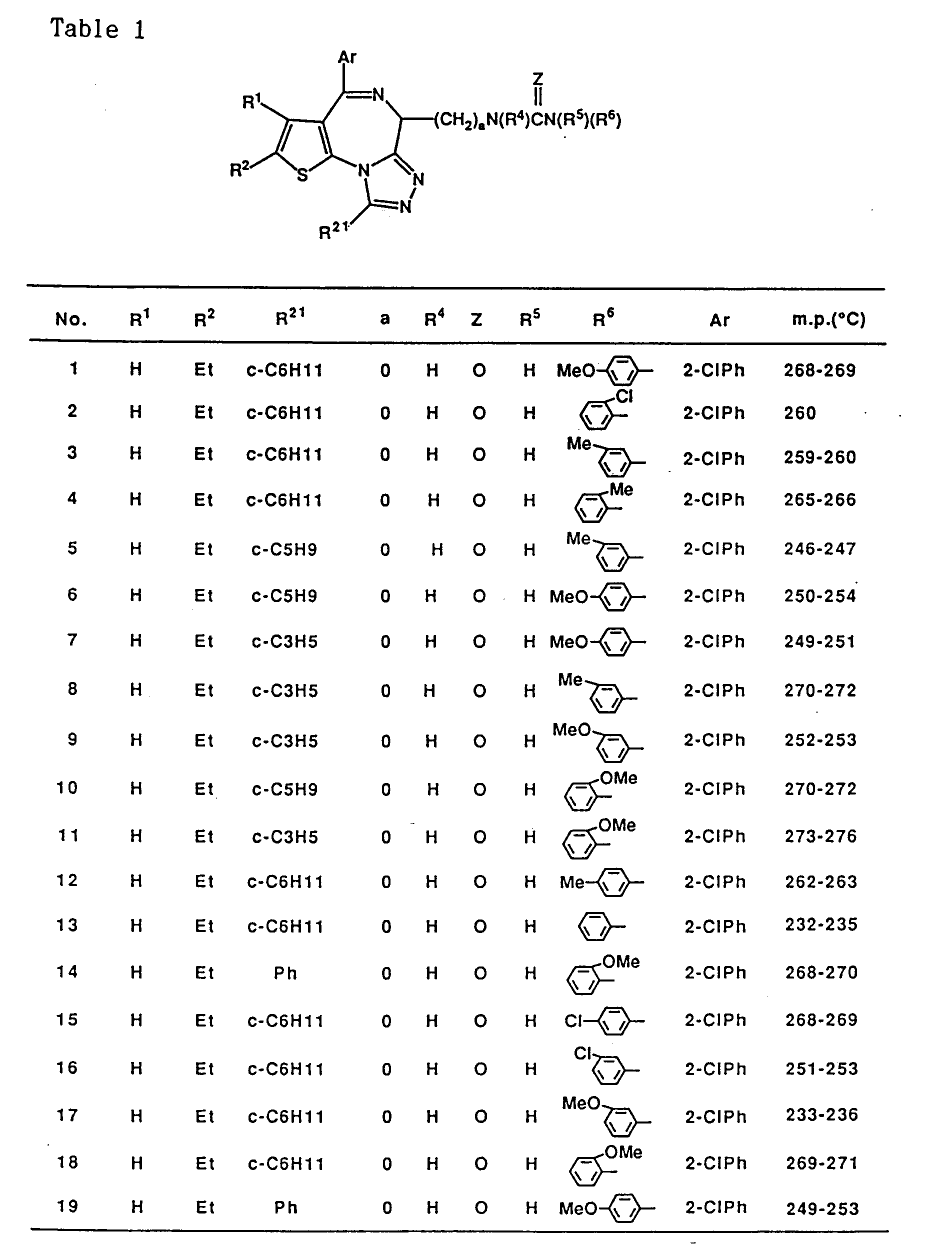

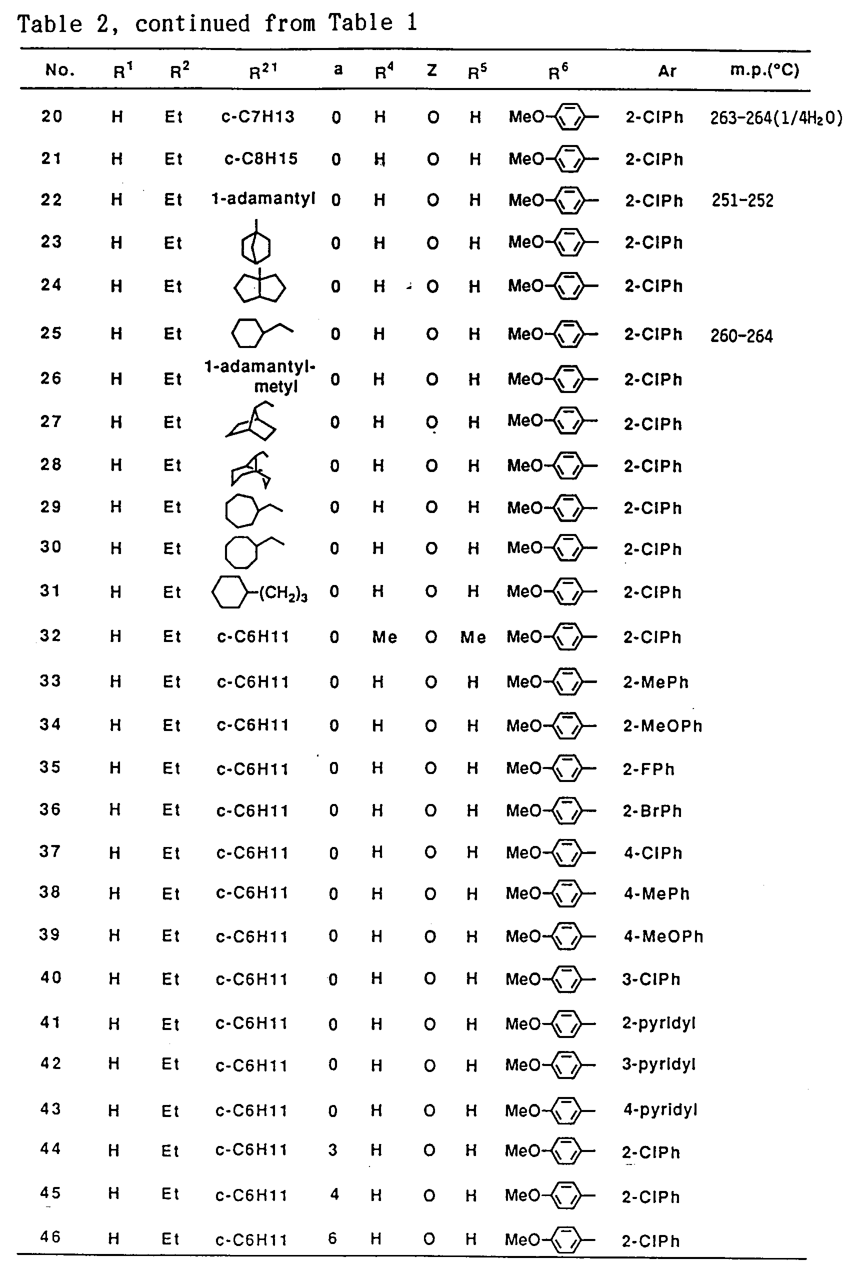

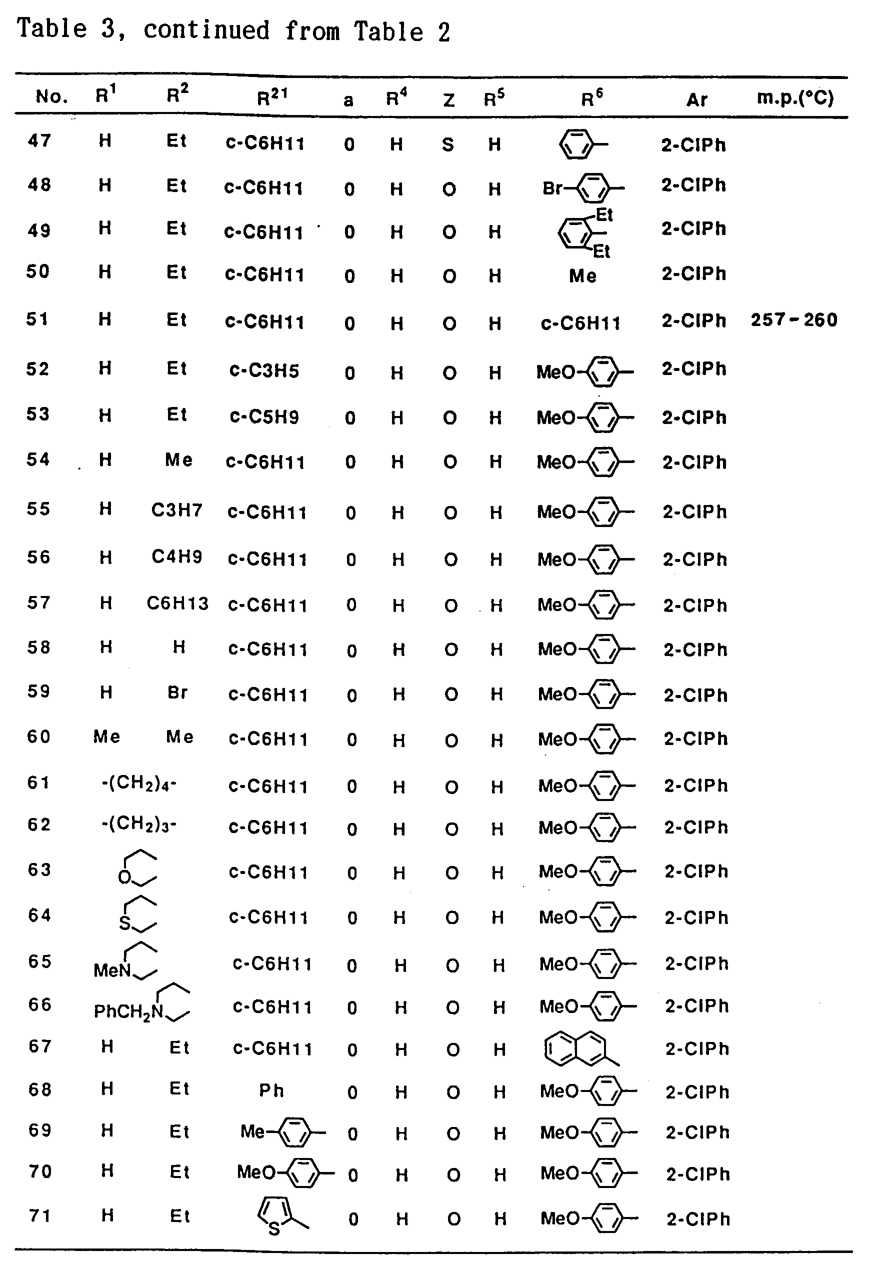

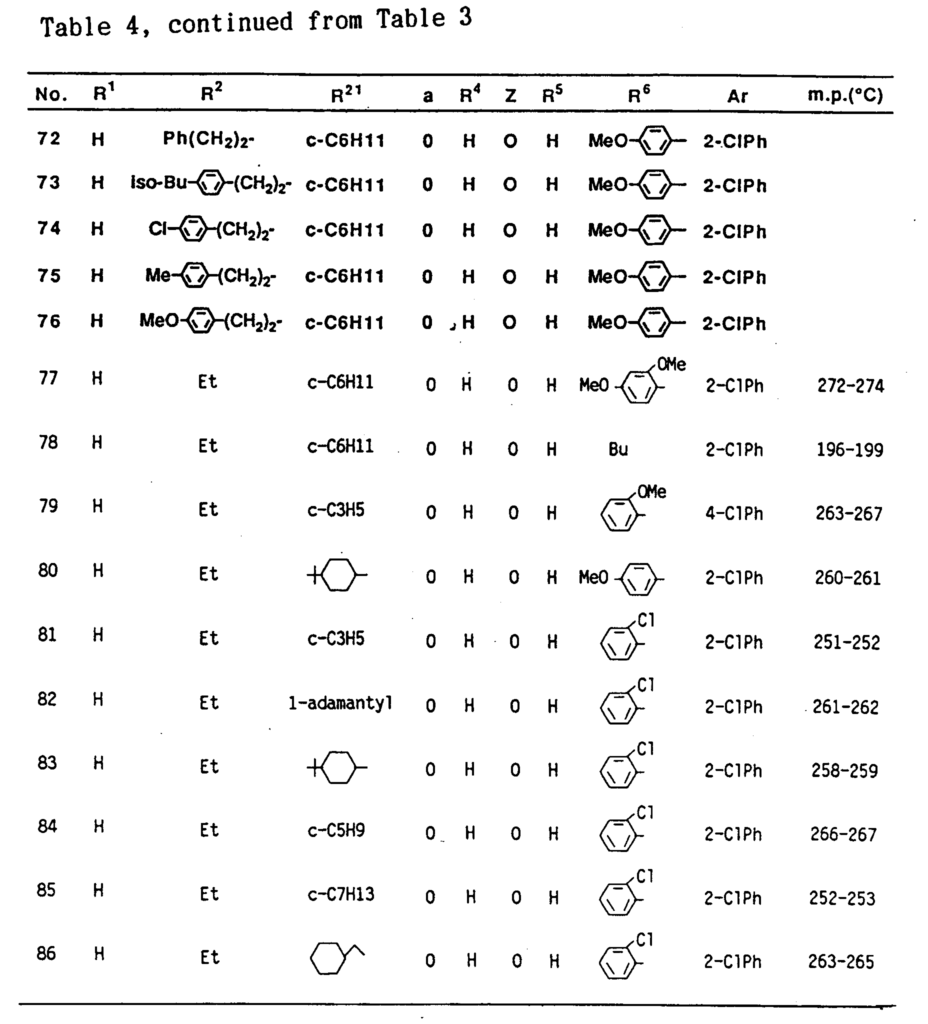

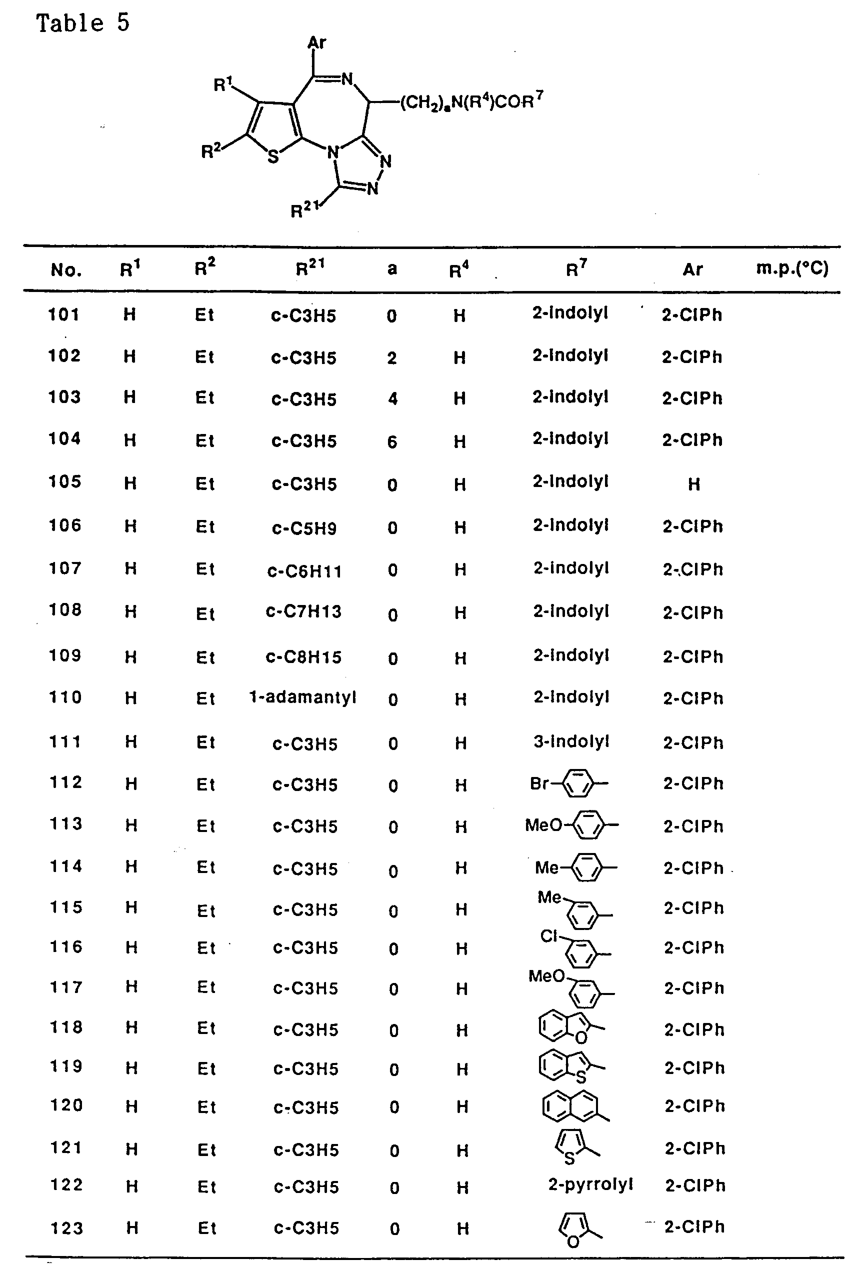

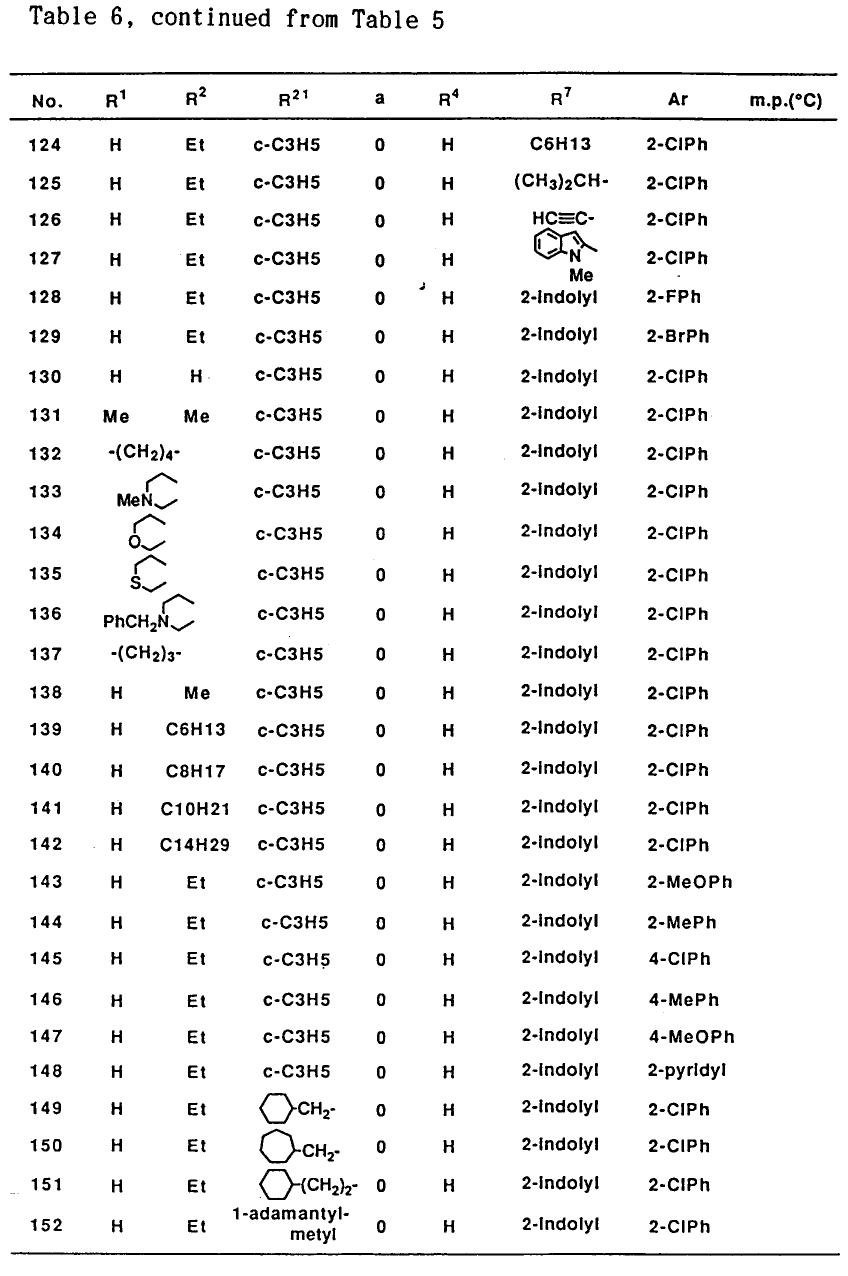

### Reference Example 1

4-(4-Chlorophenyl)-2-ethyl-9-methyl-6H-thieno[3,2-f][1,2,4]triazolo[4,3-a][1,4]diazepine (1 g) was dissolved in diethyl carbonate (35 ml) in a stream of nitrogen, and 60% sodium hydride was added with stirring at room temperature. After refluxing under heating for 2 hours, the reaction mixture was cooled to room temperature, and ethyl bromoacetate (0.32 ml) was added. After stirring at room temperature for 3 hours, the reaction mixture was poured on cool water, and extracted with ethyl acetate. After washing with water, the resulting organic layer was dried over anhydrous magnesium sulfate and filtered. The filtrate was concentrated under reduced pressure, and the residue was subjected to silica gel column chromatography. The object fraction was concentrated under reduced pressure and the residue was suspended in a mixture of ethanol (90 ml) and water (30 ml). Barium hydroxide·8 hydrate (0.65 g) was added with stirring at room temperature. After stirring at room temperature for 10 hours, the reaction mixture was concentrated under reduced pressure. Water was added and the mixture was washed with ethyl acetate. The aqueous layer was adjusted to pH 2 with 6N hydrochloric acid, and left standing at room temperature overnight. The resulting mixture was neutralized with sodium hydrogencarbonate and extracted with ethyl acetate. The extract was dried over anhydrous magnesium sulfate and filtered. The filtrate was concentrated under reduced pressure, and isopropyl ether was added to the residue to give (4-(4-chlorophenyl)-2-ethyl-9-methyl-6H-thieno[3,2-f][1,2,4]triazolo[4,3-a][1,4]diazepin-6-yl)acetic acid (0.15 g) as pale-brown crystals, melting point 198-202°C.

### Reference Example 2

In the same manner as in Reference Example 1, (5-(2-chlorophenyl)-7-ethyl-1-methyl-2-oxo-1,3-dihydro-2H-thieno[2,3-e]-1,4-diazepin-3-yl)acetic acid, melting point 228-231°C, was obtained using 5-(2-chlorophenyl)-7-ethyl-1-methyl-1,3-dihydro-2H-thieno[2,3-e]-1,4-diazepin-2-one.

### Example 201

(4-(4-Chlorophenyl)-2-ethyl-9-methyl-6H-thieno[3,2-f][1,2,4]triazolo[4,3-a][1,4]diazepin-6-yl)acetic acid (2 g) obtained in Reference Example 1 was dissolved in dimethylformamide (50 ml), and aniline (0.59 ml), triethylamine (1.4 ml) and 1-hydroxybenztriazole (0.75 g) were added with stirring at room temperature. The mixture was cooled to not more than 0°C with stirring, and N-ethyl-N'-(3-dimethylaminomethyl)carbodiimide hydrochloride (1.05 g) was added. The mixture was heated to room temperature, and allowed to stand overnight. The reaction mixture was poured on water and extracted with ethyl acetate. The organic layer was sequentially washed with 1N hydrochloric acid, 1N sodium hydroxide and water, and dried over anhydrous magnesium sulfate. After filtration, the filtrate was concentrated under reduced pressure, and the obtained crystals were recrystallized from ethanol to give 1.4 g of N-phenyl(4-(4-chlorophenyl)-2-ethyl-9-methyl-6H-thieno[3,2-f][1,2,4]triazolo[4,3- a][1,4]diazepin-6-yl)acetamide, melting point 238-239°C.

The following compounds are obtained in the same manner as above.

### Example 202

N-(3-Methylphenyl)-(4-(4-chlorophenyl)-2-ethyl-9-methyl-6H-thieno[3,2-f][1,2,4]triazolo[4,3-a][1,4]diazepin-6-yl)acetamide, melting point: 247-248°C

### Example 203

N-(3-Chlorophenyl)-(4-(4-chlorophenyl)-2-ethyl-9-methyl-6H-thieno[3,2-f][1,2,4]triazolo[4,3-a][1,4]diazepin-6-yl)acetamide, melting point: 217-218°C

### Example 204

N-(2-Methoxyphenyl)-(4-(4-chlorophenyl)-2-ethyl-9-methyl-6H-thieno[3,2-f][1,2,4]triazolo[4,3-a][1,4]diazepin-6-yl)acetamide, melting point: 198-200°C

### Example 205

N-(3-Methoxyphenyl)-(4-(4-chlorophenyl)-2-ethyl-9-methyl-6H-thieno[3,2-f][1,2,4]triazolo[4,3-a][1,4]diazepin-6-yl)acetamide, melting point: 244°C

### Example 206

6-Amino-4-(4-chlorophenyl)-2-ethyl-9-methyl-6H-thieno[3,2-f][1,2,4]triazolo[4,3-a][1,4]diazepine (1.6 g) was dissolved in chloroform (8 ml), and triethylamine (0.69 ml) was added with stirring. p-Toluenesulfonyl chloride (0.94 g) was added with stirring at room temperature, and the mixture was refluxed under heating with stirring in an oil bath for 2 hours. After cooling, the reaction mixture was concentrated under reduced pressure, and the residue was subjected to silica gel column chromatography. The object fraction was concentrated under reduced pressure, and isopropyl ether was added to the residue to crystallize 1.25 g of N-(4-(4-chlorophenyl)-2-ethyl-9-methyl-6H-thieno[3,2-f][1,2,4]triazolo[4,3-a][1,4]diazepin-6-yl)-p-toluenesulfonamide, melting point 239-242°C.

The following compound can be obtained in the same manner as above.

### Example 207

N-(4-(2-Chlorophenyl)-2-(4-isobutylphenyl)ethyl-9-methyl-6H-thieno[3,2-f][1,2,4]triazolo[4,3-a][1,4]diazepin-6-yl)-p-toluenesulfonamide, melting point: 158-162°C

### Example 208

5-(2-Chlorophenyl)-7-ethyl-1-methyl-1,3-dihydro-2H-thieno[2,3-e][1,4]diazepin-2-one (3 g) was dissolved in chloroform (60 ml), and m-chloroperbenzoic acid (4.3 g) was added with stirring at room temperature. The mixture was stirred for 8 hours. The reaction mixture was washed with 0.5N sodium hydroxide and water, and dried over magnesium sulfate. After filtration, the filtrate was concentrated under reduced pressure, and the obtained residue was dissolved in acetic anhydride (30 ml). The mixture was stirred at 70°C with heating. After cooling, the reaction mixture was poured on water, and neutralized with sodium hydrogencarbonate. The mixture was extracted with ethyl acetate. The organic layer was washed with an aqueous solution of sodium hydrogencarbonate and water, and dried over anhydrous magnesium sulfate. After filtration, the filtrate was concentrated under reduced pressure, and the residue was dissolved in methanol (40 ml). A solution of sodium hydroxide (0.6 g) in water (10 ml) was added with stirring at room temperature, and the mixture was stirred at room temperature for 3 hours. The reaction mixture was concentrated under reduced pressure, and water (50 ml) was added. The mixture was extracted with ethyl acetate, and the organic layer was dried over anhydrous magnesium sulfate. After filtration, the filtrate was concentrated under reduced pressure. The obtained residue was subjected to silica gel column chromatography to give 1.3 g of crude 5-(2-chlorophenyl)-7-ethyl-3-hydroxy-1-methyl-1,3-dihydro-2H-thieno[2,3-e][1,4]diazepin-2-one. This compound (0.7 g) was dissolved in toluene (20 ml), and 3-methylphenyl isocyanate (0.31 g) was added. The mixture was stirred at 90°C for 2 days. After cooling, the reaction mixture was concentrated under reduced pressure, and the residue was subjected to silica gel column chromatography to give 0.25 g of (5-(2-chlorophenyl)-7-ethyl-1-methyl-2-oxo-1,3-dihydro-2H-thieno[2,3-e][1,4]diazepin-3-yl)-N-(3-methylphenyl)carbamate, melting point 181-182.5°C.

The following compound can be obtained in the same manner as above.

### Example 209

4-(2-Chlorophenyl)-2-ethyl-9-methyl-6H-thieno[3,2-f][1,2,4]triazolo[4,3-a][1,4]diazepin-6-yl)-N-(3-methylphenyl)carbamate, melting point:190-192°C

The compounds shown in the following Tables can be obtained in the same manner as above.

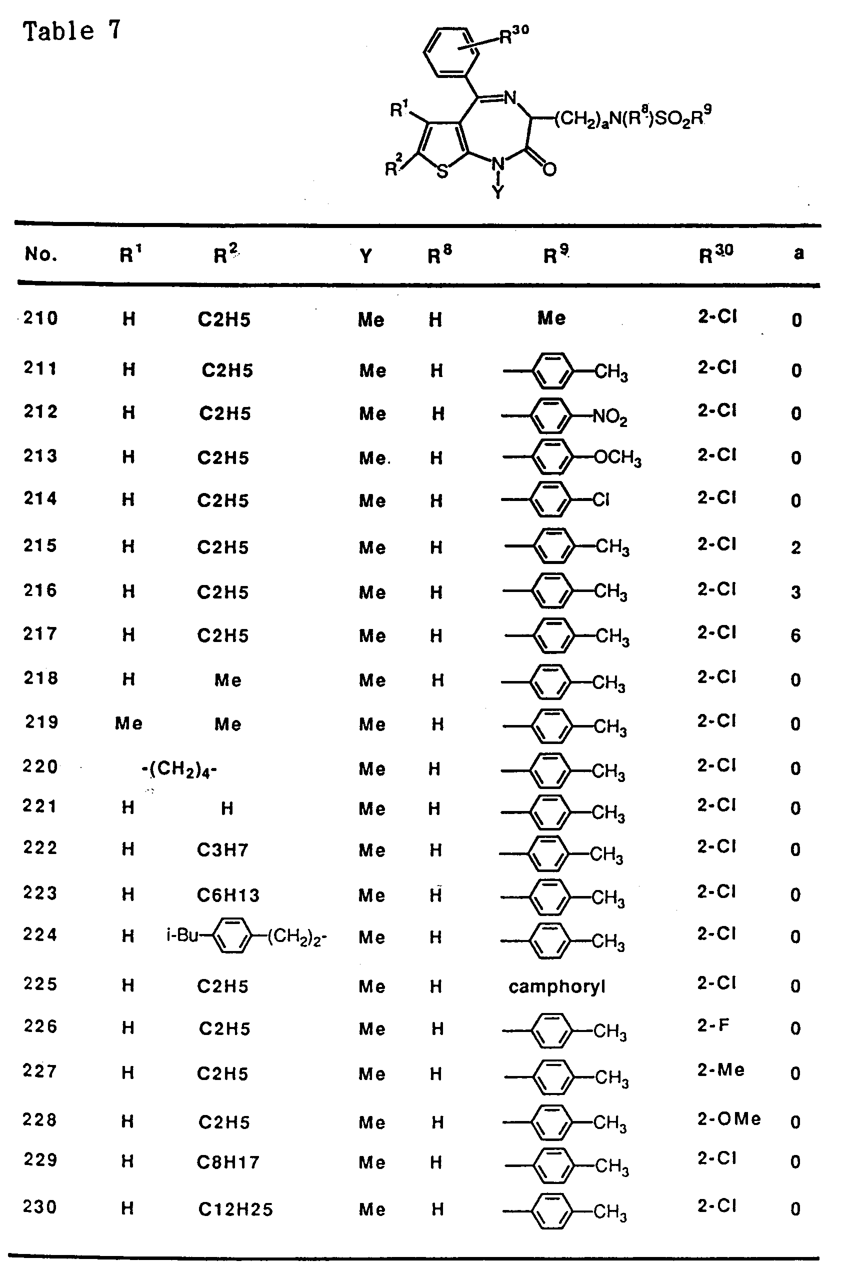

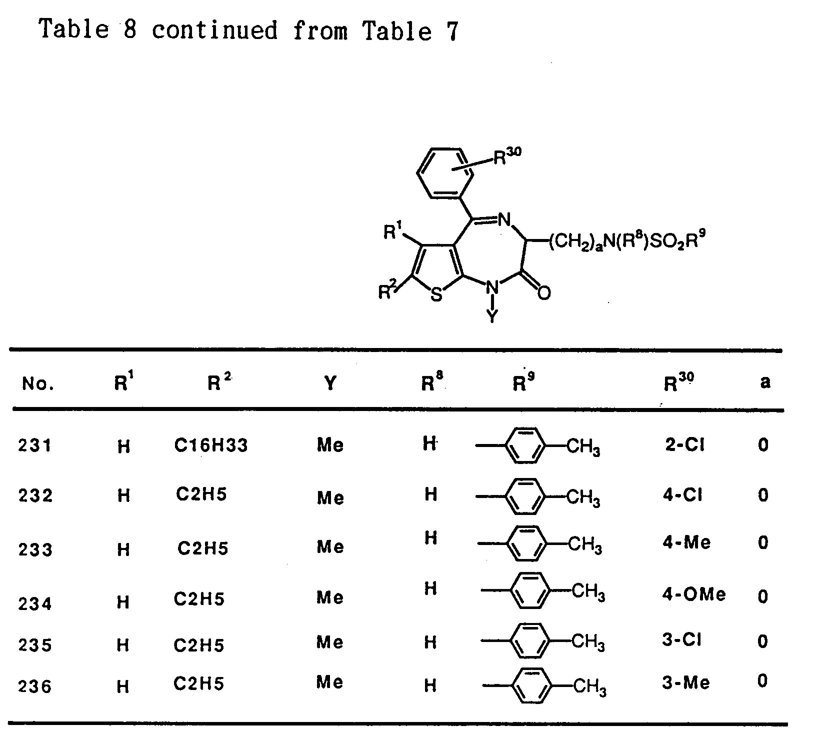

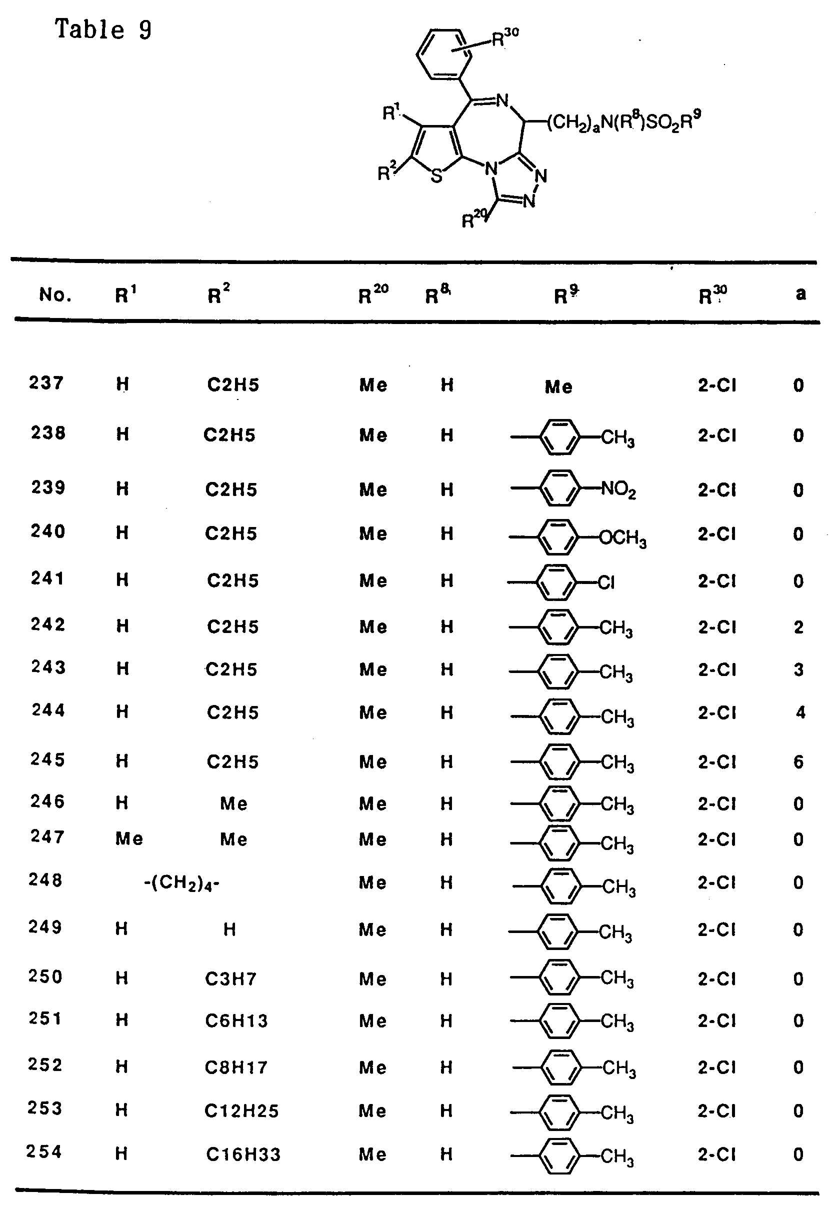

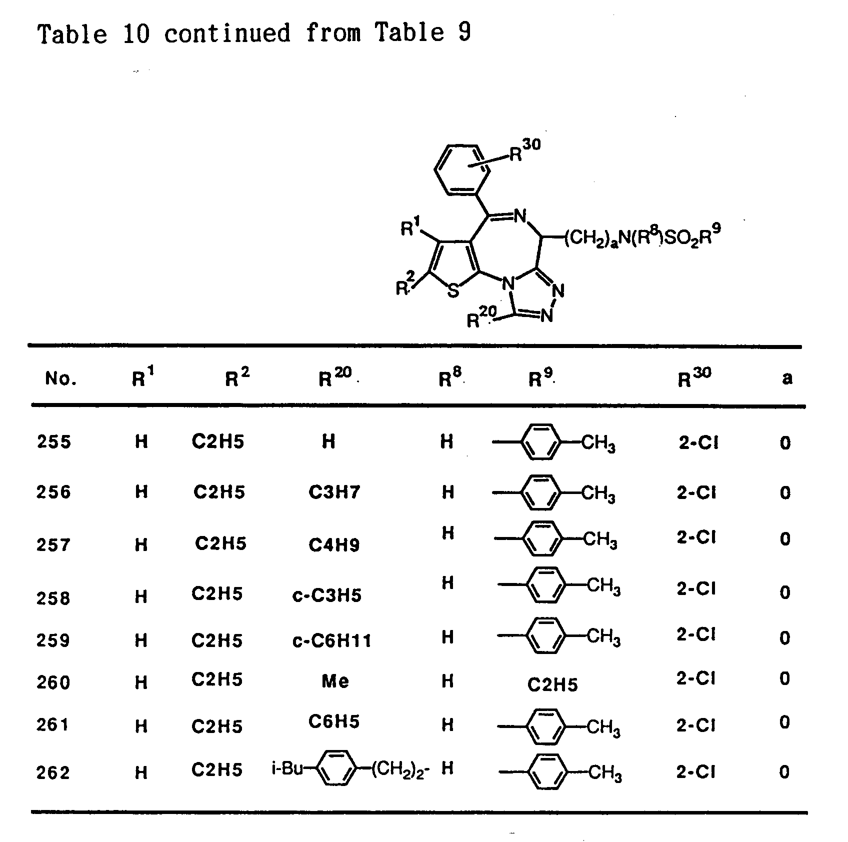

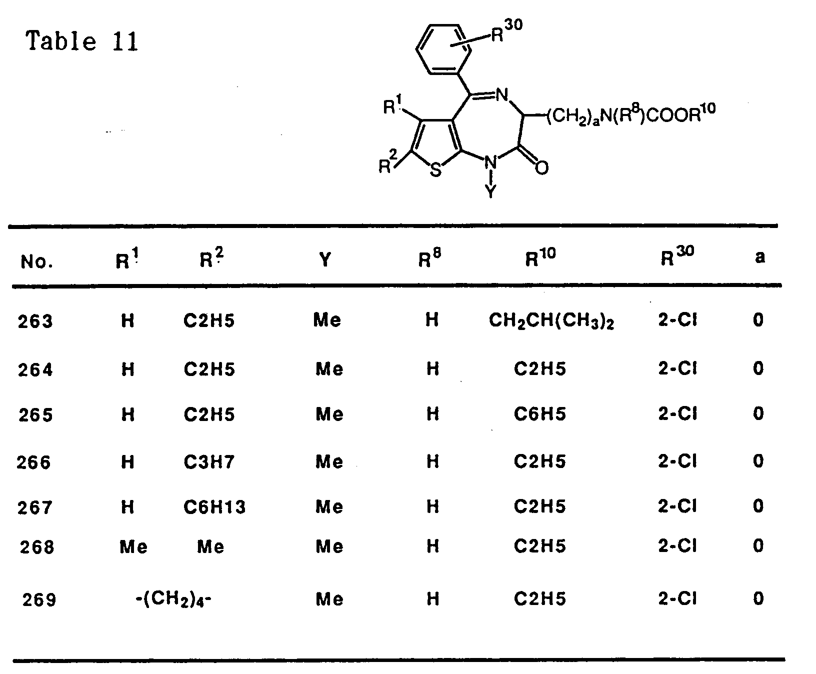

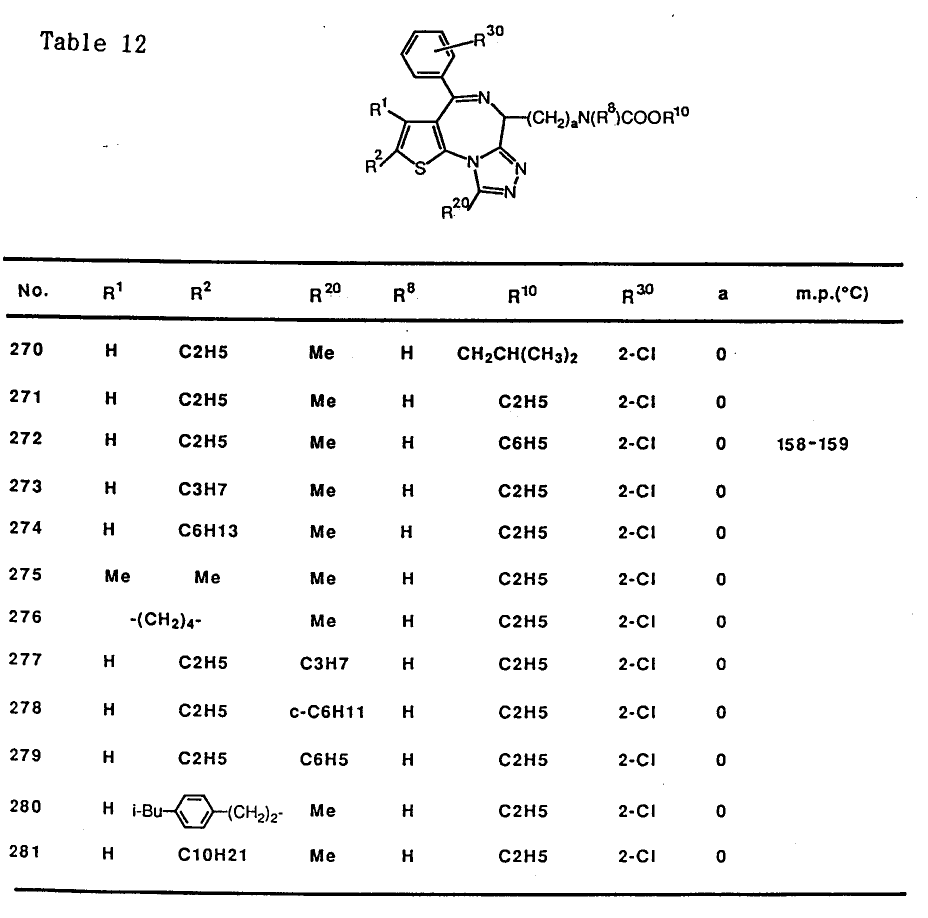

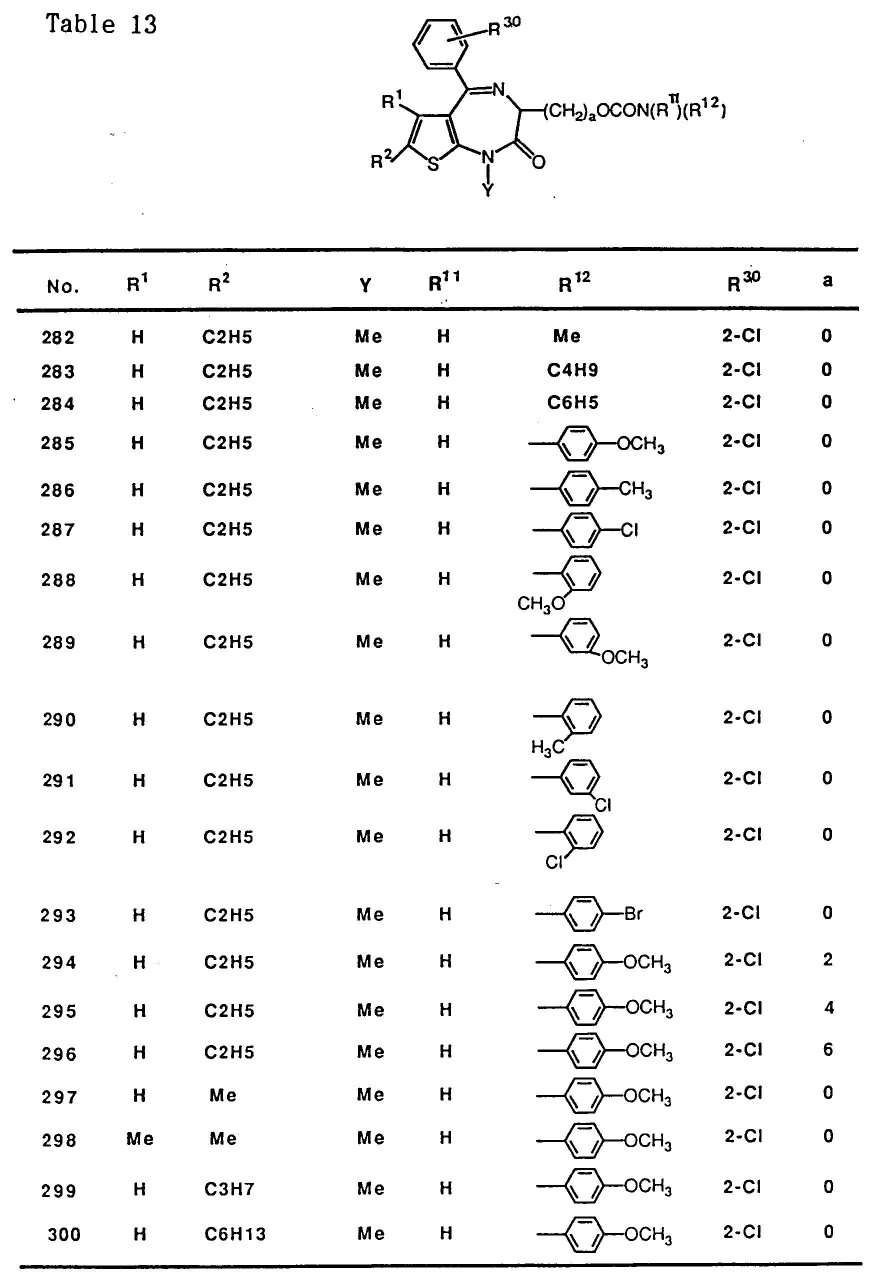

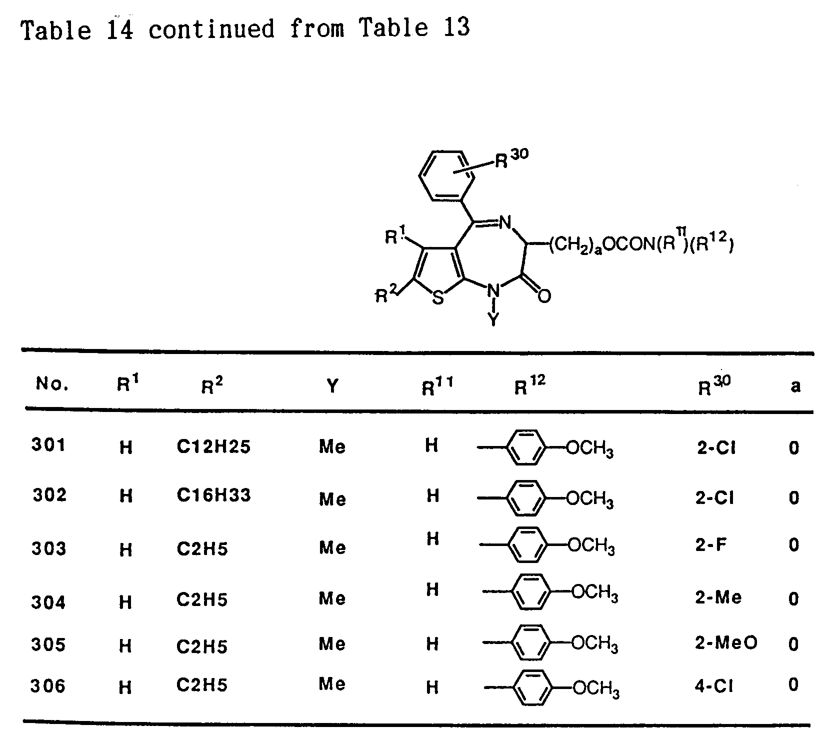

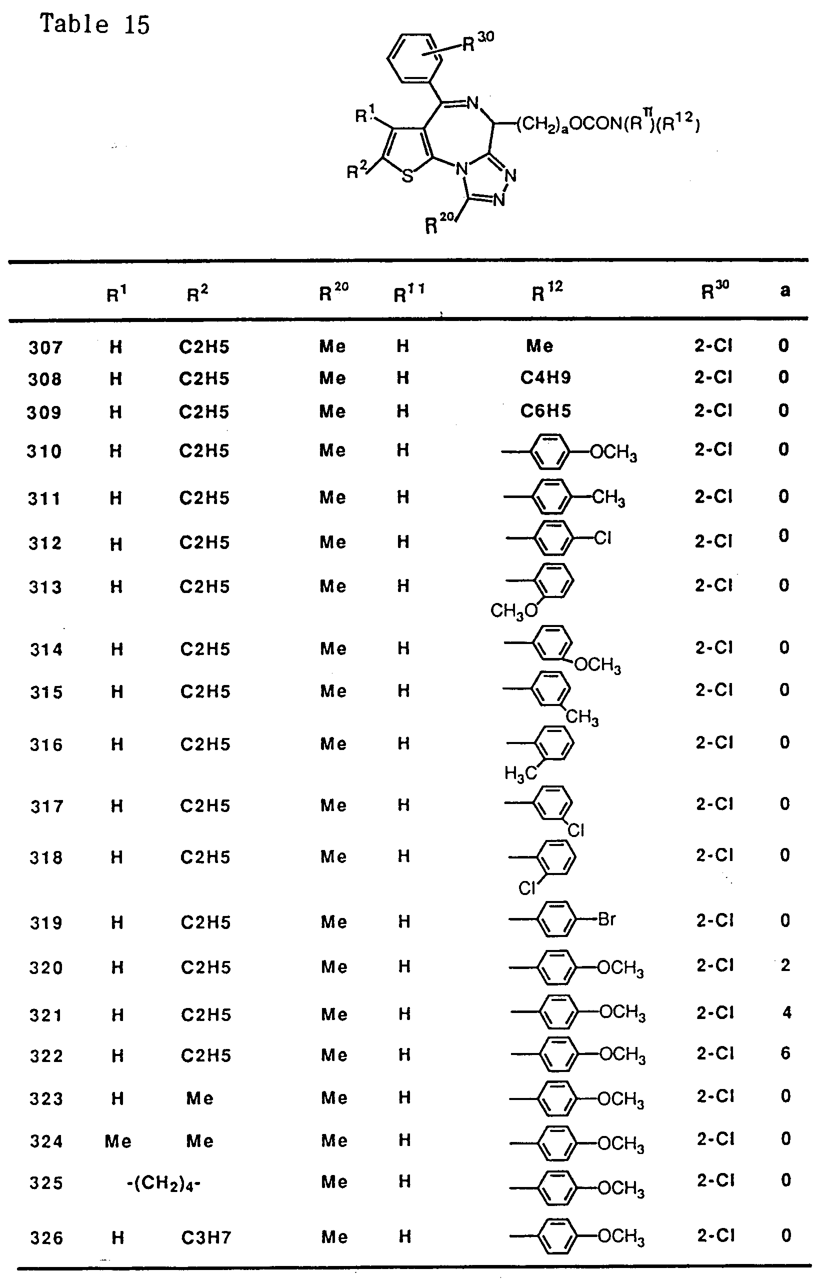

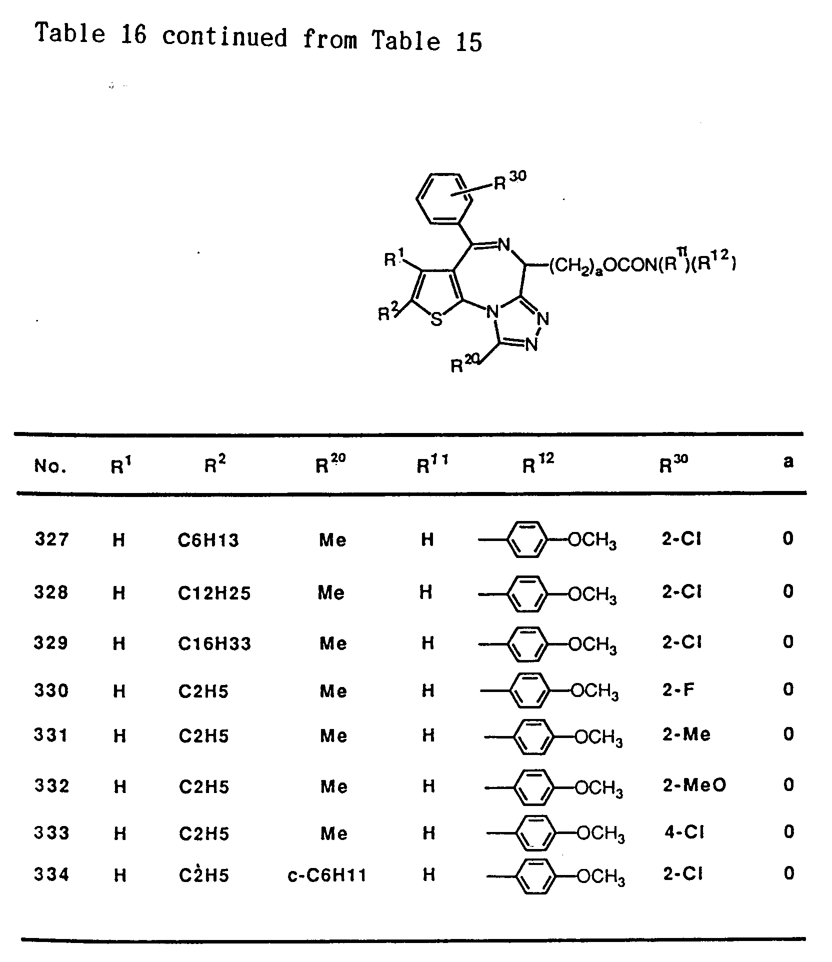

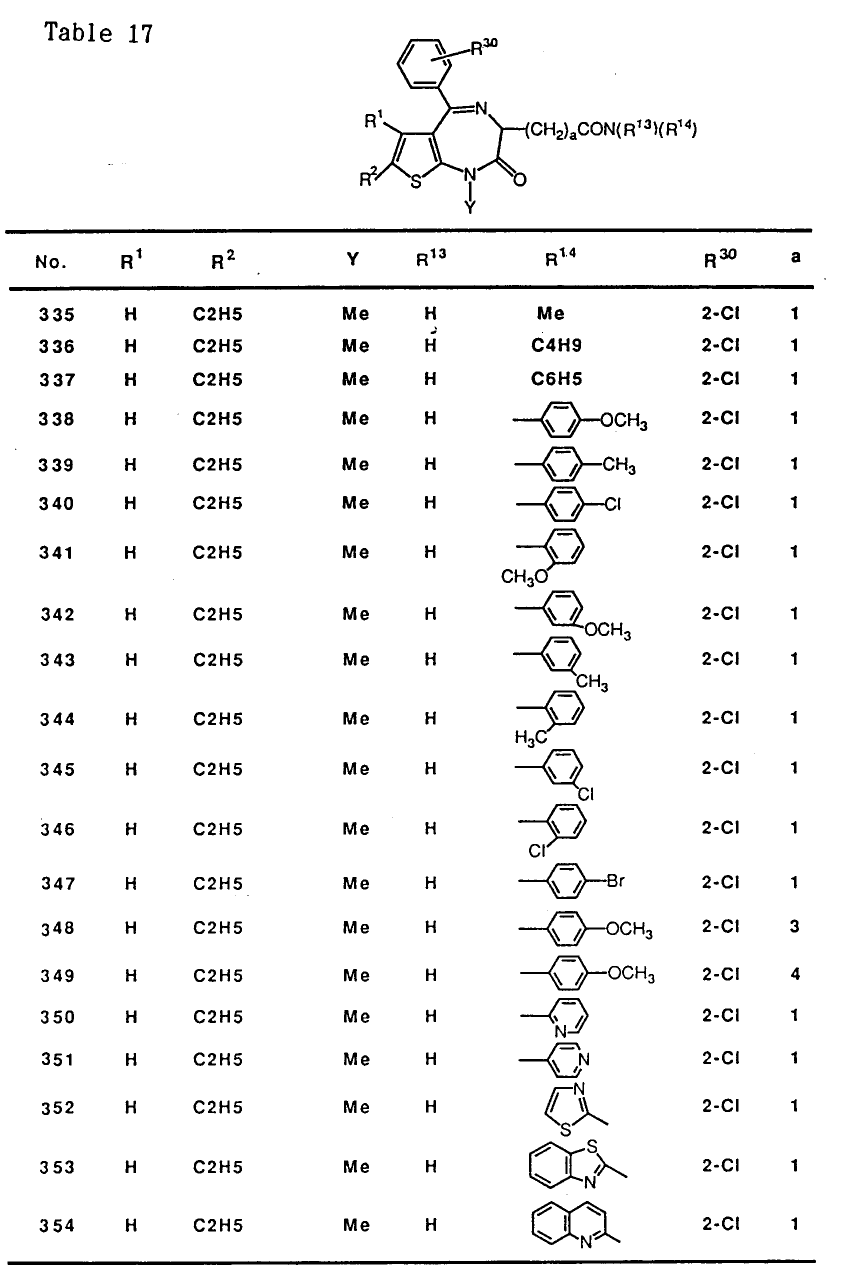

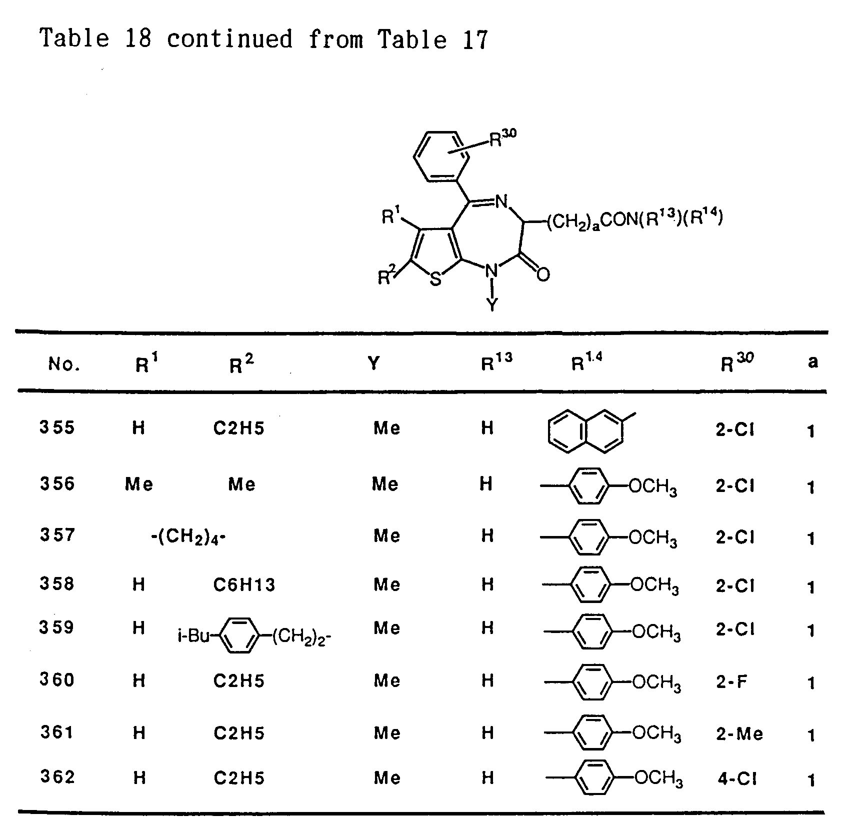

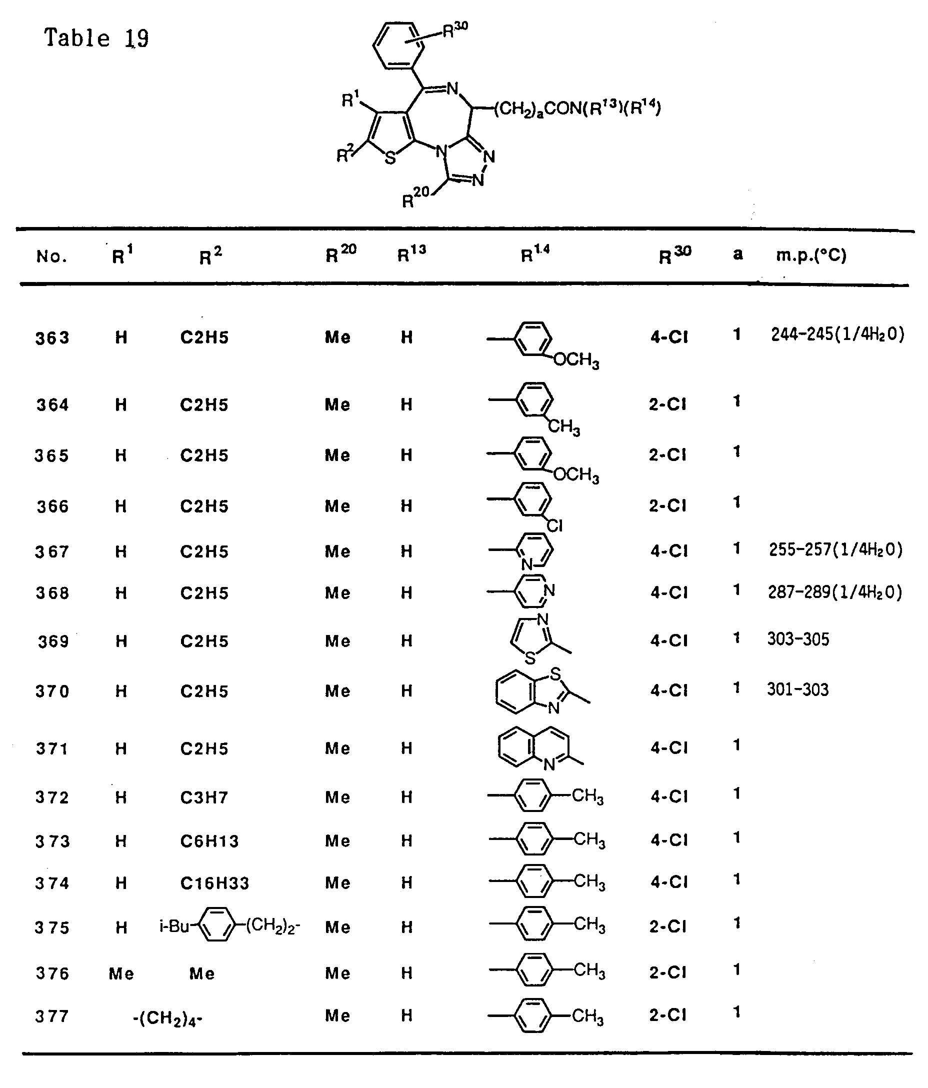

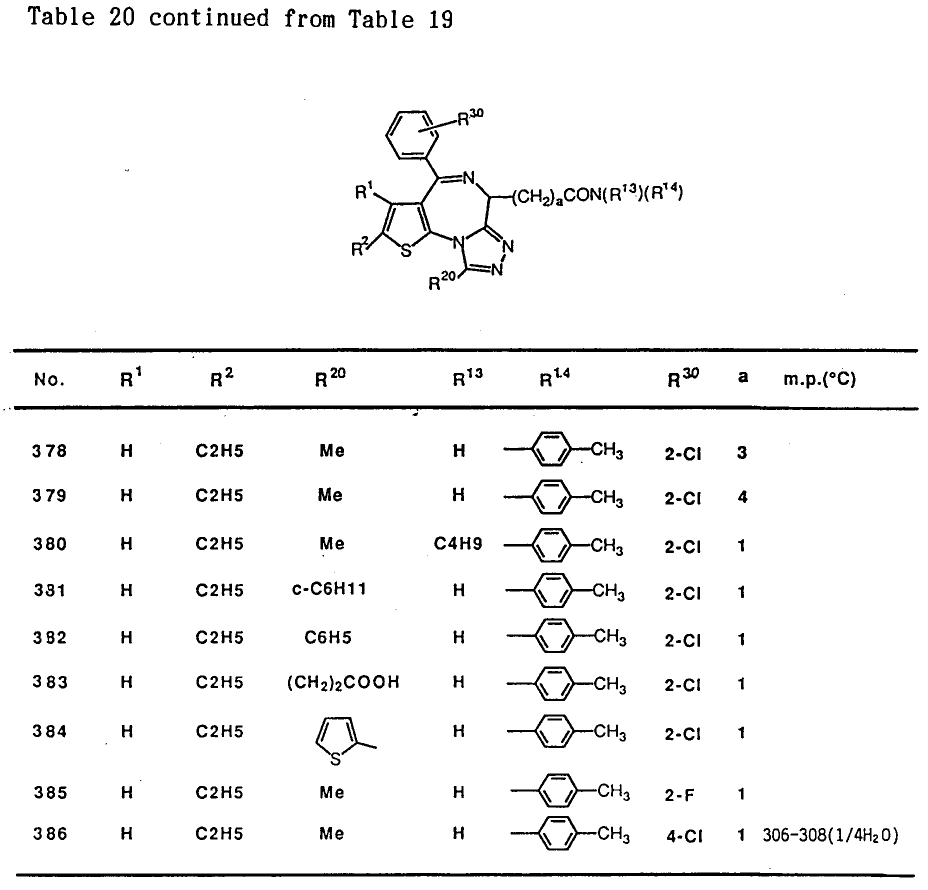

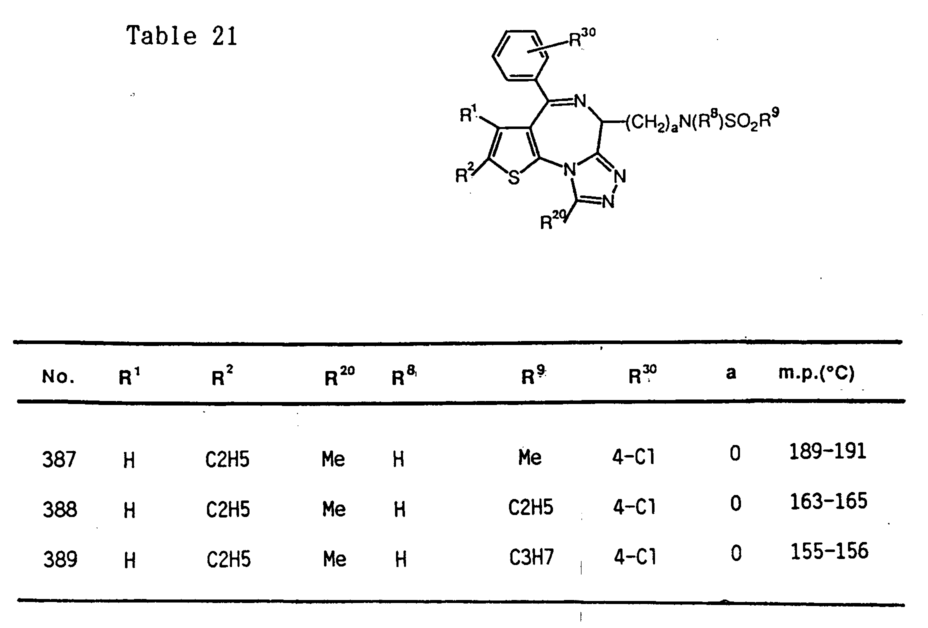

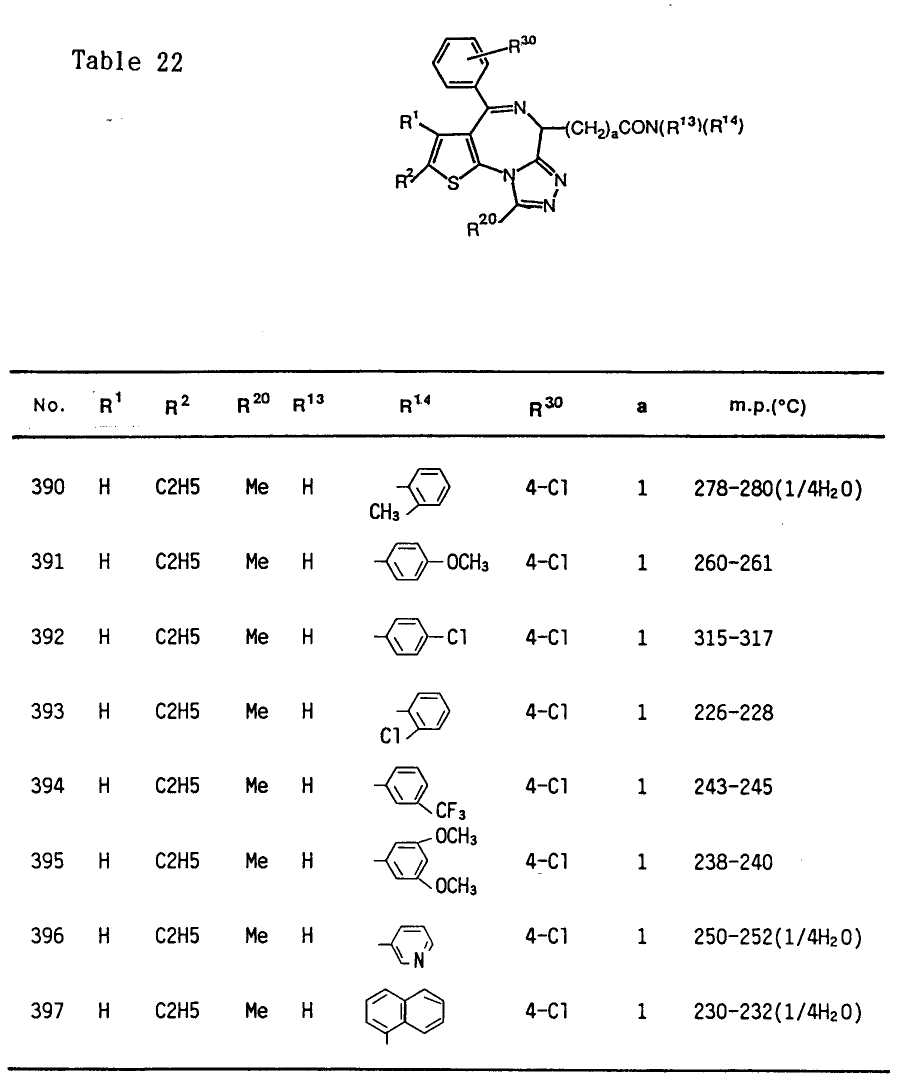

### Example 401

N-(9-(1-Adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea (250 mg) was subjected to preparative high performance liquid chromatography (HPLC, flow rate 10 ml/min) using an optical isomer separation column (manufactured by Merck, Chirasphar, inner diameter 25 mm, length 250 mm) and a mixed solution of n-hexane:dioxane:isopropyl alcohol at a volume ratio of 1800:1200:45 as a mobile phase. The fraction eluted 20 minutes later was taken and concentrated under reduced pressure to give 90 mg of (+)-N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea as a white amorphous powder.
[α] $\frac{\text{23}}{\text{D}}$ + 67.6° (c = 1, methanol)
The fraction eluted 28 minutes later was concentrated under reduced pressure to give 96 mg of (-)-N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea as a white amorphous powder.
[α] $\frac{\text{24}}{\text{D}}$ - 65.2° (c = 1, methanol)

### Formulation Example

The formulation examples of the pharmaceutical of the present invention are given in the following.

### (1) Tablet

The above-mentioned compound (I) (0.5 part), lactose (25 parts), crystalline cellulose (35 parts) and corn starch (3 parts) were thoroughly admixed, and kneaded well with a binder prepared from corn starch (2 parts). The kneaded product was passed through a 16-mesh sieve, dried in an oven at 50°C and passed through a 24-mesh sieve. The kneaded product thus obtained, corn starch (8 parts), crystalline cellulose (11 parts) and talc (9 parts) were kneaded well, compressed into tablets containing 0.5 mg of the active ingredient per tablet.

### (2) 1% Powder

The above-mentioned compound (I) (1 part) and lactose (90 parts) were thoroughly admixed, and kneaded well with a binder prepared from a suitable amount of methylcellulose. The kneaded product was passed through a 16-mesh sieve and dried in an oven at 50°C. The dried granules were forced to pass through a 32-mesh sieve, and admixed well with an appropriate amount of silicon dioxide to give a 1% powder.

## Claims

1. A thienodiazepine compound of the formula 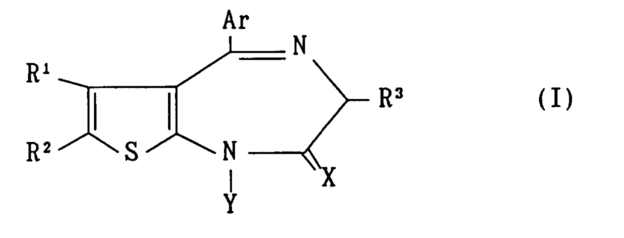 wherein
Ar is an optionally substituted aryl or an optionally substituted heteroaryl;
R¹ is a hydrogen, a halogen, an alkyl having 1 to 20 carbon atoms or an optionally substituted aralkyl;
R² is a hydrogen, a halogen, an alkyl having 1 to 20 carbon atoms, an optionally substituted aralkyl, an alkenyl having 2 to 20 carbon atoms or an alkynyl having 2 to 20 carbon atoms; or
R¹ and R² may combinedly together form an optionally substituted 5 to 7-membered ring, or an optionally substituted 5 to 7-membered heterocyclic ring together with a hetero atom;
R³ is a group of the formula 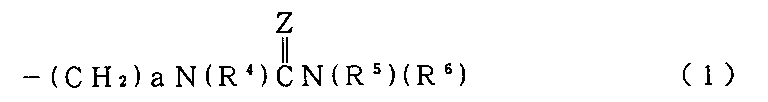
wherein a is 0 or an integer of 1-6, Z is an oxygen atom or a sulfur atom, R⁴ is a hydrogen, an alkyl having 1 to 20 carbon atoms or an optionally substituted aralkyl, R⁵ and R⁶ are the same or different and each is a hydrogen, an alkyl having 1 to 20 carbon atoms, an optionally substituted cycloalkyl, an optionally substituted cycloalkylalkyl, an optionally substituted aryl, an optionally substituted aralkyl, an optionally substituted heteroaryl or an optionally substituted heteroarylalkyl,
a group of the formula
-(CH₂)aN(R⁴)COR⁷ (2)
wherein a is 0 or an integer of 1-6, R⁴ is a hydrogen, an alkyl having 1 to 20 carbon atoms or an optionally substituted aralkyl, R⁷ is an alkyl having 1 to 20 carbon atoms, an alkenyl having 2 to 20 carbon atoms, alkynyl having 2 to 20 carbon atoms, an optionally substituted aryl, an optionally substituted heteroaryl, an optionally substituted aralkyl or an optionally substituted heteroarylalkyl, or
a group of the formula selected from the formulas:
-(CH₂)aN(R⁸)SO₂R⁹ (3)
-(CH₂)aN(R⁸)COOR¹⁰ (4)
-(CH₂)aOCON(R¹¹)(R¹²) (5)
-(CH₂)aCON(R¹³)(R¹⁴) (6)
-(CH₂)aOCOR¹⁵ (7)
-(CH₂)aOSO₂R¹⁶ (8)
-(CH₂)aCOR¹⁷ (9) and
-(CH₂)aS(O)ₙR¹⁸ (10)
wherein a is 0 or an integer of 1-6, R⁸ is a hydrogen, an alkyl having 1 to 20 carbon atoms or an optionally substituted aralkyl, R⁹ and R¹⁰ are each an alkyl having 1 to 20 carbon atoms, alkenyl having 2 to 20 carbon atoms, alkynyl having 2 to 20 carbon atoms, an optionally substituted cycloalkyl, an optionally substituted cycloalkylalkyl, an optionally substituted aryl, an optionally substituted heteroaryl, an optionally substituted aralkyl or an optionally substituted heteroarylalkyl, R¹¹, R¹², R¹³ and R¹⁴ are the same or different and each is a hydrogen, an alkyl having 1 to 20 carbon atoms, an alkenyl having 2 to 20 carbon atoms, an alkynyl having 2 to 20 carbon atoms, an optionally substituted aryl, an optionally substituted heteroaryl, an optionally substituted aralkyl or an optionally substituted heteroarylalkyl, R¹⁵, R¹⁶, R¹⁷ and R¹⁸ are each an alkyl having 1 to 20 carbon atoms, an optionally substituted aryl, an optionally substituted heteroaryl, an optionally substituted aralkyl or an optionally substituted heteroarylalkyl, and n is 0, 1 or 2;
X is an oxygen atom or a sulfur atom; and
Y is a hydrogen, an alkyl having 1 to 20 carbon atoms, an alkenyl having 2 to 20 carbon atoms, an alkynyl having 2 to 20 carbon atoms, an optionally substituted aralkyl, an optionally substituted heteroarylalkyl or a group of the formula
-(CH₂)bCOOR¹⁹
wherein R¹⁹ is a hydrogen, an alkyl having 1 to 20 carbon atoms, an alkenyl having 2 to 20 carbon atoms or an optionally substituted aralkyl and b is an integer of 1 to 6; or X and Y may combinedly form a group of the formula:
=N-N=C(R²⁰)- or =N-CH=C(R²⁰)-
wherein R²⁰ is a hydrogen, a halogen, an alkyl having 1 to 20 carbon atoms, an alkenyl having 2 to 20 carbon atoms, an alkynyl having 2 to 20 carbon atoms, an optionally substituted cycloalkyl, an optionally substituted cycloalkylalkyl, an optionally substituted aryl, an optionally substituted heteroaryl, an optionally substituted aralkyl, an optionally substituted heteroarylalkyl, or a group of the formula:
-(CH₂)bCOOR²⁰'
wherein R²⁰' is a hydrogen, an alkyl having 1 to 20 carbon atoms, an alkenyl having 2 to 20 carbon atoms or an optionally substituted aralkyl and b is an integer of 1 to 6; with the proviso that
when R³ is a group of the above-mentioned formula (1) or (2), X and Y combinedly form a group of the formula:
=N-N=C(R²¹)- or =N-CH=C(R²¹)-
wherein R²¹ is an optionally substituted cycloalkyl, an optionally substituted cycloalkylalkyl, an optionally substituted aryl, an optionally substituted heteroaryl or an optionally substituted heteroarylalkyl; and
when R³ is a group of the above-mentioned formula (7), R¹, R² and Y are each a hydrogen or an alkyl having 1 to 4 carbon atoms, X is an oxygen atom, Ar is a phenyl substituted by halogen and a is 0, R¹⁵ is an alkyl having 5 to 20 carbon atoms, an optionally substituted aryl, an optionally substituted heteroaryl, an optionally substituted aralkyl or an optionally substituted heteroarylalkyl;
or a salt thereof.

2. The compound of Claim 1, which is represented by the formula 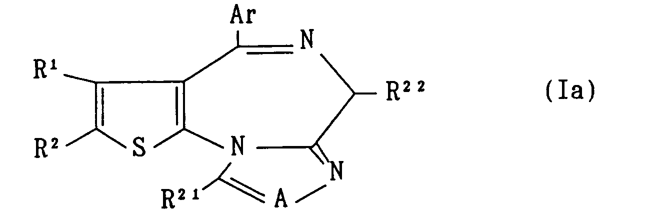 wherein Ar, R¹, R² and R²¹ are as defined in Claim 1, A is a nitrogen atom or CH and R²² is a group of the formula 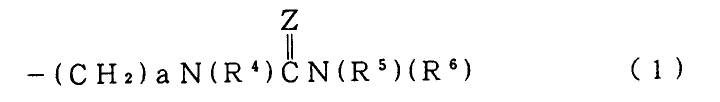 wherein each symbol is as defined in Claim 1, or a group of the formula
-(CH₂)aN(R⁴)COR⁷ (2)
wherein each symbol is as defined in Claim 1, or a salt thereof.

3. The compound of Claim 1, which is represented by the formula 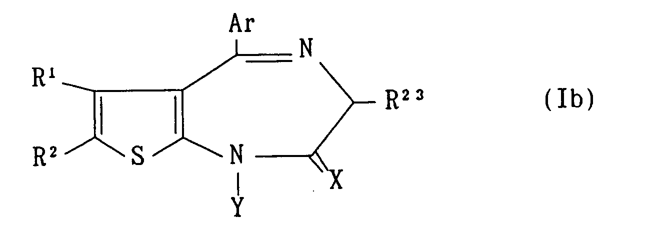 wherein Ar, R¹, R², X and Y are as defined in Claim 1 and R²³ is a group of the formula selected from the group consisting of:
-(CH₂)aN(R⁸)SO₂R⁹ (3)
-(CH₂)aN(R⁸)COOR¹⁰ (4)
-(CH₂)aOCON(R¹¹)(R¹²) (5)
-(CH₂)aCON(R¹³)(R¹⁴) (6)
-(CH₂)aOCOR¹⁵ (7)
-(CH₂)aOSO₂R¹⁶ (8)
-(CH₂)aCOR¹⁷ (9) and
-(CH₂)aS(O)ₙR¹⁸ (10)
wherein each symbol is as defined in Claim 1, provided that when R²³ is a group of the above-mentioned formula (7), R¹, R² and Y are each a hydrogen or an alkyl having 1 to 4 carbon atoms, X is an oxygen atom, Ar is a phenyl substituted by halogen and a is 0, R¹⁵ is an alkyl having 5 to 20 carbon atoms, an optionally substituted aryl, an optionally substituted heteroaryl, an optionally substituted aralkyl or an optionally substituted heteroarylalkyl, or
a salt thereof.

4. The thienodiazepine compound of Claim 1 or Claim 2, wherein the compound of the formula (I) is selected from the group consisting of;
N-(4-(2-chlorophenyl)-9-cyclohexyl-2-ethyl-6H-thieno[3,2-f][1,2,4]triazolo[4,3-a][1,4]diazepin-6-yl)-N'-(4-methoxyphenyl)urea,
N-(2-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(4-(2-chlorophenyl)-9-cyclohexyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(2-methylphenyl)urea,
N-(4-(2-chlorophenyl)-9-cyclopentyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclopentyl-2-ethyl-6H-thieno[3,2-f][1,2,4]triazolo[4,3-a][1,4]diazepin-6-yl)-N'-(4-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(4-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(3-methylphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(3-methoxyphenyl)urea,
N-(4-(2-chlorophenyl)-9-cyclopentyl-2-ethyl-6H-thieno[3,2-f][1,2,4]triazolo[4,3-a][1,4]diazepin-6-yl)-N'-(2-methoxyphenyl)urea,
N-(4-(2-chlorophenyl)-9-cyclopropyl-2-ethyl-6H-thieno[3,2-f][1,2,4]triazolo[4,3-a][1,4]diazepin-6-yl)-N'-(2-methoxyphenyl) urea,
N-(4-(2-chlorophenyl)-9-cyclohexyl-2-ethyl-6H-thieno[3,2-f][1,2,4]triazolo[4,3-a][1,4]diazepin-6-yl)-N'-(4-methylphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(phenyl)urea,
N-(4-(2-chlorophenyl)-2-ethyl-9-phenyl-6H-thieno[3,2-f][1,2,4]triazolo[4,3-a][1,4]diazepin-6-yl)-N'-(2-methoxyphenyl)urea,
N-(4-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(3-chlorophenyl)-N'-(4-(2-chlorophenyl)-9-cyclohexyl-2-ethyl-6H-thieno[3,2-f][1,2,4]triazolo[4,3-a][1,4]diazepin-6-yl)urea,
N-(4-(2-chlorophenyl)-9-cyclohexyl-2-ethyl-6H-thieno[3,2-f][1,2,4]triazolo[4,3-a][1,4]diazepin-6-yl)-N'-(3-methoxyphenyl)urea,
N-(4-(2-chlorophenyl)-9-cyclohexyl-2-ethyl-6H-thieno[3,2-f][1,2,4]triazolo[4,3-a][1,4]diazepin-6-yl)-N'-(2-methoxyphenyl)urea,
N-(4-(2-chlorophenyl)-2-ethyl-9-phenyl-6H-thieno[3,2-f][1,2,4]triazolo[4,3-a][1,4]diazepin-6-yl)-N'-(4-methoxyphenyl)urea,
N-(4-(2-chlorophenyl)-9-cycloheptyl-2-ethyl-6H-thieno[3,2-f][1,2,4]triazolo[4,3-a][1,4]diazepin-6-yl)-N'-(4-methoxyphenyl)urea,
N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea,
N-(9-(1-adamantyl)-4-(2-chlorophenyl)-2-ethyl-6H-thieno[3,2-f][1,2,4]triazolo[4,3-a][1,4]diazepin-6-yl)-N'-(2-chlorophenyl)urea and
(+)-N-(9-(1-adamantyl)-4-(2-chlorophenyl)-2-ethyl-6H-thieno[3,2-f][1,2,4]triazolo[4,3-a][1,4]diazepin-6-yl)-N'-(4-methoxyphenyl)urea, or
a salt thereof.

5. The thienodiazepine compound of Claim 1 or Claim 3, wherein the compound of the formula (I) is selected from the group consisting of;
N-phenyl-(4-(4-chlorophenyl)-2-ethyl-9-methyl-6H-thieno[3,2-f][1,2,4]triazolo[4,3-a][1,4]diazepin-6-yl)acetamide,
N-(3-methylphenyl)-(4-(4-chlorophenyl)-2-ethyl-9-methyl-6H-thieno[3,2-f][1,2,4]triazolo[4,3-a][1,4]diazepin-6-yl)acetamide,
N-(3-chlorophenyl)-(4-(4-chlorophenyl)-2-ethyl-9-methyl-6H-thieno[3,2-f][1,2,4]triazolo[4,3-a][1,4]diazepin-6-yl)acetamide,
N-(2-methoxyphenyl)-(4-(4-chlorophenyl)-2-ethyl-9-methyl-6H-thieno[3,2-f][1,2,4]triazolo[4,3-a][1,4]diazepin-6-yl)acetamide,
N-(3-methoxyphenyl)-(4-(4-chlorophenyl)-2-ethyl-9-methyl-6H-thieno[3,2-f][1,2,4]triazolo[4,3-a][1,4]diazepin-6-yl)acetamide,
N-(4-(4-chlorophenyl)-2-ethyl-9-methyl-6H-thieno[3,2-f][1,2,4]triazolo[4,3-a][1,4]diazepin-6-yl)-p-toluenesulfonamide,
N-(4-(2-chlorophenyl)-2-(4-isobutylphenyl)ethyl-9-methyl-6H-thieno[3,2-f][1,2,4]triazolo[4,3-a][1,4]diazepin-6-yl)-p-toluenesulfonamide,
(5-(2-chlorophenyl)-7-ethyl-1-methyl-2-oxo-1,3-dihydro-2H-thieno[2,3-e][1,4]diazepin-3-yl)-N-(3-methylphenyl)carbamate and (4-(2-chlorophenyl)-2-ethyl-9-methyl-6H-thieno[3,2-f][1,2,4]triazolo[4,3-a][1,4]diazepin-6-yl)-N-(3-methylphenyl)carbamate,
or
a salt thereof.

6. A pharmaceutical composition comprising a therapeutically effective amount of the compound of Claim 1, 2, 3, 4 or 5, and a pharmaceutically acceptable additive.

7. A thienodiazepine compound of the formula (II) 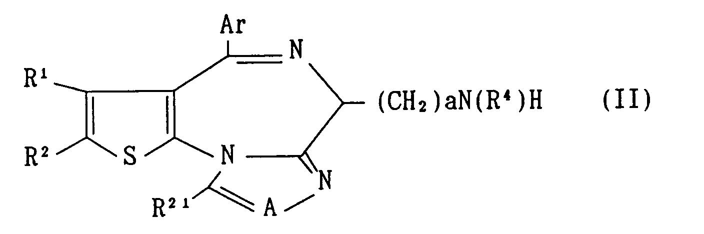 wherein A is a nitrogen atom or CH and other symbols are as defined in Claim 1, or
a salt thereof.
